# EUROPEAN PATENT APPLICATION

(11) **EP 4 191 787 A1**
(43) Date of publication of application: **07.06.2023**
(21) Application number: 21315268.9
(22) Date of filing: 06.12.2021
(51) Int. Cl.: H01Q 1/27, A61N 5/04

(54) **ENERGY MANAGEMENT FOR MILLIMETRIC MODULE**

(71) Applicant: Remedee Labs, 38330 Montbonnot Saint Martin (FR)
(72) Inventor: Crouzier, David, 38240 Meylan (FR); Foerster, Michael, 38700 Corenc (FR)
(74) Representative: Schuffenecker, Thierry

(57) **Abstract**

There is described a system comprising a millimeter waves emitter, with one or more frequencies selected in the band 61.0 - 61.5 GHz and a power flux density of approximately 10 mW/cm², connectable to a source of energy. Radiation doses can reduce chronic pain, of low to moderate intensity. Developments describe the use of logic circuit(s), sensors and/or actuators, the use of a second energy source, possibly releasable. Various options are described: the connection between the emitter and/or one or more energy sources can be wired but also wireless, e.g. performed by power beaming. Object tracking device can be used. Different types of batteries can be used, and energy can even be drained from available consumer electronics devices. Magnetic connections can be used, along fast-charge batteries or various communication networks and protocols.

## Description

### Technical field

The document provides examples of a millimetric module configured to irradiate mammals for the treatment of pain, in particular the treatment of chronic pain of low to moderate intensity.

### Background

Some people suffer from chronic pain. Chronic pain is recurrent, or even continuous, and can occur for months or years, without the origin being necessarily determined or eliminated. The most common approach to treating a patient's pain is to administer so-called analgesic doses. However, regular intake of such analgesic active ingredients can cause undesirable side effects (for example: nausea, drowsiness, heartburn). An alternative to taking medication is to treat the pain by physical methods. The risk of side effects is then reduced, as well as that of dose dependence. There is a known form of treatment by transcutaneous electrical neurostimulation (or TENS for "Transcutaneous electrical nerve stimulation") which consists in circulating an electric current in an area of the patient's body to stimulate nerves in order to reduce pain. The use of this technique generally requires a bulky device. Lastly, it is a known fact that therapy by transmission of so-called "millimeter" waves (whose frequency is less than 300 gigahertz) reduces pain. It has been shown that the exposure of an area of the human body to millimeter electromagnetic waves allowed the release of endogenous opioids, generating in the brain the synthesis of enkephalin, a natural peptide involved in pain tolerance. This makes it possible to avoid the disadvantages of therapies with analgesics or by neurostimulation, and its modus operandi is understood. In addition, transmitting millimeter waves can also be used in the treatment of anxiety or sleep disorders.

Application WO2012/022538 discloses a device aimed at reducing pain in a patient by transmitting electromagnetic waves to the surface of the patient's body. The device is bulky and presents other limitations.

### Summary of the invention

There is described a system comprising a millimeter waves emitter, with one or more frequencies selected in the band (from) 61.0 (up to) 61.5 GHz and a power flux density of approximately 10 mW/cm², connectable to a source of energy. Radiation doses can reduce chronic pain, of low to moderate intensity. Developments describe the use of logic circuit(s), sensors and/or actuators, the use of a second energy source, possibly releasable. Various options are described: the connection between the emitter and/or one or more energy sources can be wired but also wireless, e.g. performed by power beaming. Object tracking device can be used. Different types of batteries can be used, and energy can even be drained from available consumer electronics devices. Magnetic connections can be used, along fast-charge batteries or various communication networks and protocols.

Associated advantages and/or technical effects can be described. Millimeter waves (MMW) stimulate subcutaneous nerve receptors of the wrist (or other radiation location), sending a message to the brain, which in turn releases endorphins. Endorphins are natural opioids and their release varies throughout the day and according to the stimulation received by the peripheral nervous system. Thus, painful (e.g. nociceptive stimuli, pregnancy and childbirth and non-painful, massage, light stimuli can lead to an increase in endorphin levels. Endorphins are involved in pain regulation both by reducing ascending transmission of the nociceptive message and by descending inhibition from the brain to the spinal cord. At the peripheral level, endogenous opioids reduce the ascending transmission of nociceptive impulses. At the central level, endogenous opioids reduce interneural signal transmission of the nociceptive message and inhibit the release of GABA, thus resulting in abundant release of dopamine. Dopamine is a main actor in the feelings of pleasure, reward and euphoria and as such modulates the perception of pain, especially its affective and motivational aspects. Endogenous opioids also influence the balance between sympathetic and parasympathetic systems namely by inhibition of the β-adrenergic activity which results in a modulation of breathing and heartbeat. In addition, endogenous opioids also regulate the cholinergic activity, which is strongly involved in one's level of arousal. At the behavioral level, the release of endorphins triggers sleep onset.

As an electronic medication relying on one's own resource, there's no risk of overdose, nor any other side effects. As a wearable device, the therapeutic wristband is a "at home" treatment which can be used at convenience. The patients are thus completely autonomous and responsible for their own treatment in terms of time and frequency.

Advantageously, systems according to the invention can treat pain effectively by means of electromagnetic waves, and to make the devices provided for this purpose accessible and easily usable by patients.

Advantageously, systems according to the invention can be used for generation of a feeling of well-being, the treatment of anxiety or even sleep disorders.

Advantageously, the device is able to be worn by a human being at least in one of the following sites: around a wrist; on a leg; on an ankle; in the back; on an ear; in the palm of a hand; or more generally any site presenting a strongly innervated area:

### Brief description of drawings

Embodiments of the present invention will now be described by way of example with reference to the accompanying drawings in which like references denote similar elements, and in which:
FIG. 1 illustrates an embodiment of the system according to the invention;
FIG. 2 illustrates an embodiment of the system according to the invention.

### Detailed description

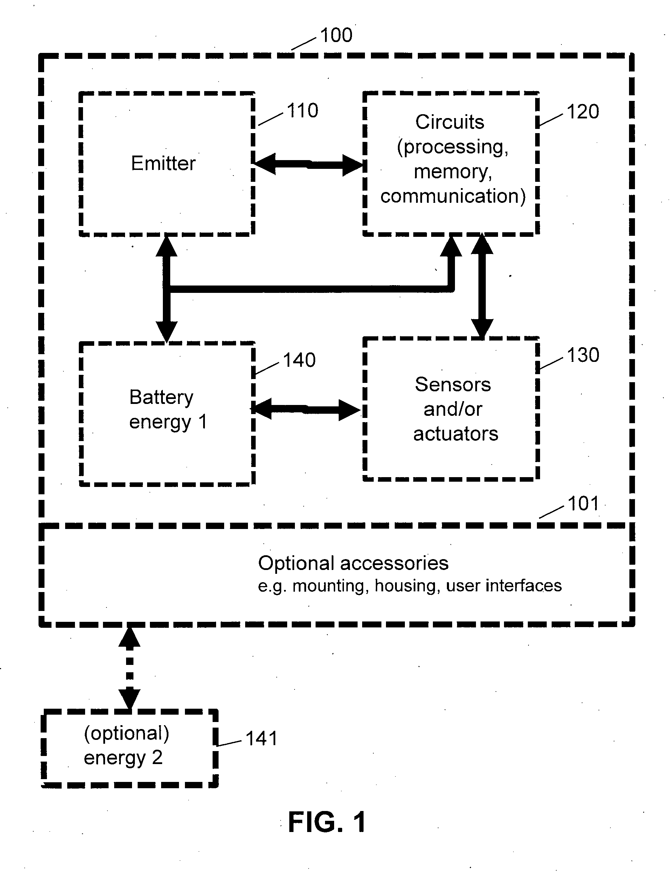

FIG 1 shows an embodiment of the invention.

In an embodiment, the system 100 according to the invention comprises an emitter 110. The emitter can be controlled by a logic circuit 120. Optionally, the emitter 110 is connectable to a battery or other energy source 130, according to different variants (e.g. permanent or releasable). Optionally, the emitter (actuator) 110 and/or the logic 120 is associated with different sensors 140. The system 100 is associated with one or more accessories 101, for example mounting systems (e.g. wristbands, armbands, necklace). Optionally, an energy source 141 can be temporarily connected to the system 100.

### Emitter 110

Electromagnetic waves allow the transport of energy. When they are applied to biological tissue, they are absorbed by the tissue they pass through to a maximum depth where all their energy has been dissipated. The maximum depth reached depends on their physical characteristics and the tissues they pass through. The energy absorbed by the tissue is transformed either into heat or chemical or electrical modification. Wavelength and electrical properties of the tissue are the key parameter of the depth penetration, and where the energy carried by the electromagnetic field is deposed.

Devices in accordance with embodiments of the present disclosure, emit (e.g., through antennas) millimeter wave radiation of about 61.25 GHz, plus or minus 250 MHz, at emitted power densities on the order of 15 mW/cm2. Portions of the device also may increase in temperature, through the power consumption of the electronic components mounted on an electronic printed circuit incorporated therewith.

At frequencies around 60 GHz, electromagnetic waves emitted by devices in accordance with embodiments of the present disclosure may be able to penetrate bare human skin to depths of about 0.5 to 1 millimeter with about 90% of the power being absorbed in the epidermis and the dermis layer

Most of the energy may be absorbed specifically at the location of fibroblasts in the skin, and at the stratum basal layer of the epidermis.

Alternatively, the module may be capable of discontinuously exposing 2.5 cm2 to waves, that is to say, several surface portions distributed over several different locations which, all together, represent 2.5 cm2 of simultaneously irradiated surface.

It should be noted that the frequency transmitted by the different antennas is not necessarily the same. The different antennas may transmit different frequencies, being supplied by different ASICs. Nevertheless, the frequencies remain within the band of interest.

Advantageously, the waves have a power flux density of between 5 and 35 mW/cm2.

Thus, it is a particularly efficient power band. On the other hand, some standards require an upper limit for the emitted power density (e.g. 35mW/cm2), so that the device may be calibrated so as not to exceed this limit, if necessary.

### Accessories 101, e.g. heat sink

In an embodiment, the system according to the invention comprises a heat sink comprising at least one of the following elements: a flexible material; a phase change material; a thermal buffer; graphite; and an elastomeric material.

In an embodiment, the system further comprises one or more user interfaces. The interface can be a graphical User Interface (U.I.), in 2D (display screen) and/or in 3D (e.g. augmented and/or virtual reality), with or without haptic input and/or output devices. The UI also can comprise or be performed by audio (sounds, music, etc.), vibrations, odors or others (nervous influx, electrical signal, etc.).

Wristbands can be hydrophobic. Wristbands can comprise an inflatable part to protect it from clothes and/or to maintain appropriate distance between the emitter and the skin of the user.

### Logic circuit 120

The driving parameters of the emitter may be set by an external microcontroller or logic that is capable of adjusting the ratio conducted heat/radiated RF energy, and total energy produced.

In an embodiment, a millimeter module can further comprise a rechargeable battery.

Preferably, the device assembly comprising the module, has a battery supplying both the control module and the wave transmission module.

In an embodiment, the battery can be recharged. It is interesting that its autonomy is several hours, even several days, so that the patient's portable device aimed at treating his pain is more convenient to use.

Some of the module components can be placed outside thereof to better interact with the device comprising the module, such as the battery.

A module (less than 3 cubic centimeters) can transmit electromagnetic waves and is suitable, when it is arranged at a surface, to transmit electromagnetic waves having a power flux density of at least 0.5 milliwatts per square centimeter of surface (the minimum power flux density of 0.5 mW/cm² is that from which millimeter wave treatment is effective for treating pain, but this module may be intended for other uses.

### Energy source 140

In some embodiments, a single battery or energy source 140 is necessary and sufficient. For example it can be placed in or near the millimetric module (a housing can comprise the millimetric emitter 110, the logic circuit or processor 120, the optional sensors 130 and the energy source 140).

In an optional embodiment, a second battery or energy source 141 can be (at least temporarily) connected to the system 100. This additional battery can be used to deliver electromagnetic doses.

### Regulations of the emitter e.g. by the logic circuit

In some embodiments, regulation can be closed-loop (such as an "artificial" or "automated" pancreas) or open-loop (with user intervention, e.g. at least confirmation).

In an embodiment, the system according to the invention can further comprise one or more logic circuits configured to control and/or to interact with one or more of said sensors and/or actuators.

Logic circuits (i.e. hardware) embody (e.g. "realize" or "implement") software. The relationship can be unidirectional ("control", e.g. in one of the two directions) or can be bidirectional ("interaction", e.g. with feedback-loop, with feedforward mechanisms, etc.)

The term "processor" designates one or more of a general-purpose microprocessor (e.g., a central processing unit (CPU)), a graphics processing unit (GPU), a microcontroller, a Digital Signal Processor (DSP), an Application Specific Integrated Circuit (ASIC), a Field Programmable Gate Array (FPGA), a Programmable Logic Device (PLD), a controller, a state machine, gated logic, discrete hardware components, or any other suitable entity that can perform calculations or other' manipulations of information. A processor can be multi-core or many-core.

Computing processes and/or threads can be discriminated, for example according to their nature. A grade or score for example can be associated with a degree of medical priority. Priority schemes can be complex in the very details. For example, the granularity of computing and/or storage requirements can depend or be a function of or adjust latency, bandwidth, CPU core (e.g. load, parking, stability, etc), caching, performance, etc.

A watchdog or daemon can monitor the appropriate functions of the system and raise alarms if applicable. Algorithmic complexity of code can be estimated. The controllability of loosely-coupled sensors subsystems can be monitored.

### Sensors and/or actuators 130

The system can comprise one or more sensors and/or actuators. One or more of these sensors can be separate components or be comprised within other subassemblies, e.g. the ASIC emitter, or the microcontroller.

A proximity sensor for example can be used. In an embodiment, at 3 millimeters or less, wave transmission can be allowed (if the skin is not detected, for example if the distance between the device and the skin is excessive, then no transmission takes place).

The system can comprise a diversity of other sensors and/or actuators

Embodiments of the invention can comprise one or more sensors, selected from the group comprising a geophone, hydrophone, microphone, position sensor, air-fuel ratio meter, blind spot monitor, crankshaft position sensor, curb feeler, defect detector, temperature sensor, ECT sensor temperature sensor, Hall effect sensor, pressure sensor, flow sensor, oxygen sensor, parking sensor, speedometer, speed sensor, reluctance sensor, Breathalyzer, Carbon dioxide sensor, Carbon monoxide detector, Chemical field-effect transistor, Electrochemical gas sensor, Electronic nose, Electrolyte-insulator-semiconductor sensor, Fluorescent chloride sensor, Holographic sensor, Hydrocarbon dew point analyzer, Hydrogen sensor, Hydrogen sulfide sensor, Infrared point sensor, Ion-selective electrode, Nondispersive infrared sensor, Microwave chemistry sensor, Nitrogen oxide sensor, Olfactometer, Optode, Oxygen sensor, Ozone monitor, Pellistor, pH glass electrode, Potentiometric sensor, Redox electrode, Zinc oxide nanorod sensor, Current sensor, Electroscope, Galvanometer, Hall effect sensor, Hall probe, Magnetic anomaly detector, Magnetometer, microelectromechanical systems (MEMS), magnetic field sensor, Metal detector, Planar Hall sensor, Radio direction finder, Voltage detector, Actinometer, Bedwetting alarm, Ceilometer, Dew warning, Electrochemical gas sensor, Fish counter, Frequency domain sensor, Gas detector, Hook gauge evaporimeter, Humistor, Hygrometer, Leaf sensor, Pyranometer, Pyrgeometer, air flow meter, liquid flow meter, anemometer, mass flow sensor, Water meter, Bubble chamber, , Altimeter, Attitude indicator, Depth gauge, Fluxgate compass, Gyroscope, Inertial navigation system, Inertial reference unit, Magnetic compass, Ring laser gyroscope, Turn coordinator, Variometer, Vibrating structure gyroscope, Yaw rate sensor, Accelerometer, Auxanometer, Capacitive displacement sensor, Capacitive sensing, Free fall sensor, Gravimeter, Gyroscopic sensor, Inclinometer, Integrated circuit piezoelectric sensor, Laser rangefinder, Laser surface velocimeter, LIDAR, Linear encoder, Linear variable differential transformer (LVDT), Liquid capacitive inclinometers, Odometer, Photoelectric sensor, Piezoelectric accelerometer, Position sensor, Rate sensor, Rotary encoder, Rotary variable differential transformer, Tilt sensor, Tachometer, Ultrasonic thickness gauge, Variable reluctance sensor, Velocity receiver, Charge-coupled device, Colorimeter, Contact image sensor, Electro-optical sensor, Flame detector, Infra-red sensor, Kinetic inductance detector, LED, light sensor, Light-addressable potentiometric sensor, Nichols radiometer, Fiber optic sensor, Optical position sensor, Photodetector, Photodiode, Photomultiplier tubes, Phototransistor, Photoelectric sensor, Photoionization detector, Photomultiplier, Photoresistor, Photo switch, Phototube, Scintillometer, , Barograph, Barometer, Piezometer, Pirani gauge, Pressure sensor, Pressure gauge, Tactile sensor, Time pressure gauge, Hydrometer, Force gauge, Level sensor, Load cell sensor, Magnetic level gauge, Piezoelectric sensor, Strain gauge, Torque sensor, Viscometer, Bolometer, Bimetallic strip, calorimeter, Heat flux sensor, Infrared thermometer, , Resistance temperature detector, Resistance thermometer, Silicon bandgap temperature sensor, Temperature gauge, Thermistor, Thermocouple, Thermometer, Pyrometer, Alarm sensor, Doppler radar, Motion detector, Occupancy sensor, Proximity sensor, Passive infrared sensor, Reed switch, Stud finder, , Wired glove, Active pixel sensor, Back-illuminated sensor, Biochip, Biosensor, Capacitance probe, Catadioptric sensor, Carbon paste electrode, Digital sensor, Displacement receiver, Electromechanical film, Electro-optical sensor, Fabry-Perot interferometer, Fisheries acoustics, Image sensor, Image sensor format, Inductive sensor, Lab-on-a-chip, Leaf sensor, Machine vision, Micro-sensor arrays, Photoelasticity, Quantum sensor, RADAR, Ground-penetrating radar, Synthetic aperture radar, Radar tracker, SONAR, Staring array, Transducer, Ultrasonic sensor, Video sensor, Visual sensor network, Wheatstone bridge, Wireless sensor network, Actigraphy, Analog image processing, Atomic force microscopy, Catadioptric sensor, Chemoreceptor, Compressive sensing, Cryogenic particle detectors, Dew warning, Diffusion tensor imaging, Digital holography, Electronic tongue, Fine Guidance Sensor, Flat panel detector, Functional magnetic resonance imaging, Glass break detector, Heartbeat sensor, Hyperspectral sensor, biosensor, Interferometric Reflectance Imaging Sensor, Laser beam profiler, Millimeter wave scanner, Magnetic resonance imaging, Moire reflectometry, Molecular sensor, Nano sensor, Nano-tetherball Sensor, Omnidirectional camera, Optical coherence tomography, Positron emission tomography, Push broom scanner, sensitive air-conductivity sensor, Range imaging, Structured-light 3D scanner, nanowire single-photon detector, Thin-film thickness monitor, Time-of-flight camera, TriDAR, etc.

An actuator operates in the "reverse" direction of a sensor: it takes an electrical input and turns it into physical action.

Actuators used in embodiments the invention can be diverse. Types of actuators comprise linear actuators, rotary actuators, hydraulic actuators, pneumatic actuators, electric actuators, thermal and magnetic actuators mechanical actuators, supercoiled polymer actuators.

In some embodiments, an actuator can be a drug delivery device. In some embodiments, an actuator can be a buzzer (vibratile element, to transmit information such as a confirmation to the user). An actuator can be a miniaturized ventilator or heat dissipation mechanism. In some embodiments, an actuator can be an inflatable piece which can help maintaining appropriate distance from the emitter to the skin, even during movements or gestures of the user. Preceding embodiments can be combined.

In some embodiments, an actuator can be a micro actuator. A micro actuator is generally a microscopic servomechanism that supplies and transmits a measured amount of energy for the operation of another mechanism or system. Micro actuators can be based on three-dimensional mechanical structures with very small dimensions which are produced with the help of lithographic procedures and non-isotropic etching techniques. For an actuator-like displacement the most different principles of force generation are used, such as the bimetal effect, piezo effect, shape memory effect and electrostatic forces.

Micro actuators can comprise one or more of a micromotor, positioning system, gripper system, microoptics, microscanner, microchopper, microvalves, micropumps,

An actuator can be used for obtaining one or more of a comb drive, hydraulic piston, electric motor, relay, thermal bimorph, digital micromirror device, piezoelectric actuator, electroactive polymer, servomechanism, etc.

Shape memory polymer (SMP) actuators can provide a response to a range of stimuli such as light, electrical, magnetic, heat, pH, and moisture changes.

3D printed actuators can be used. Polymers such as dielectric elastomers, ionic polymer metal composites, ionic electroactive polymers, polyelectrolyte gels, and gel-metal composites can form 3D layered structures that can work as soft actuators.

A dose delivery device can use one or more shock absorbers (serial or parallel arrangements), in order to smooth the impact of the movements of the patient. Shock absorption can be passive but also active if not reactive or adaptative (MEMS or actuators can counterbalance mechanical constraints). *A contrario* skin or tissue massage (facilitating the diffusion of dose and/or analyte) can be used with similar electro-mechanical miniaturized devices.

An actuator for example can be a buzzer (vibratile element), a heat sink, an inflatable part to maximize the contact between skin and emitter, a skin or tissue massage mechanism via electro-mechanical miniaturized devices, etc.

In advantageous embodiments, sensors are medical sensors (or assimilated), or otherwise linked to the body of the patient (dermal temperature, heart rate variability, lung volume sensor, heart fibrillation sensor, epilepsy detector, tremor measurement sensor, etc.)

In some embodiments, a plurality of sensors can be used to determine various emotional states, and/or physical conditions. For example, a stress state can be determined from the detection of perspiration (skin humidity sensor), heart rate variability, respiration rhythm, movements of parts of the body (e.g. legs, vision by eye-tracking) etc. Other unusual states (e.g. depressive state, anxiety, stress, fatigue, headache, exhaustion, etc.). Heuristics, logic (e.g. fuzzy logic), rules or machine learning hyperparameters can be used to determine said states, with various probability or scales/scores.

An accelerometer can be used to recognize and monitor body posture, such as sitting, kneeling, crawling, laying, standing, walking and running. Such ability can be essential to many applications, including virtual reality, healthcare, sports and electronic games. The accelerometer-based posture monitoring for Body Area Networks typically consists of 3-axis accelerometers (or tri-axial accelerometers) which can be placed on some strategic locations on a human body. They can also be used to measure the vibration, as well as acceleration due to the gravity. A gyroscope can be used for measuring or maintaining orientation, based on the principle of conservation of angular momentum. Gyroscopes can be used together with accelerometers for physical movement monitoring. One or more accelerometers can quantify ' the physiological and/or emotional state of the patient (e.g. level of pain, anxiety, stress...).

The system according to the invention may measure, calculate, or use a plurality of other physiological metrics in addition to, or in place of, the user's inputs (e.g. relative to perceived pain). These include, but are not limited to, caloric energy expenditure, floors climbed or descended, heart rate, heart rate variability, heart rate recovery, location and/or heading (e.g., through GPS), elevation, ambulatory speed and/or distance traveled, swimming lap count, bicycle distance and/or speed, blood pressure, blood glucose, skin conduction, skin and/or body temperature, electromyography data, electroencephalographic data, weight, body fat, and respiration rate.

In addition or in substitution, external or environmental parameters can be taken into account: for example air pollution levels, atmospheric conditions (e.g. for arthritis), political climate, pandemic figures, periods of the year (e.g. holidays), etc. More generally, big data approaches (enabled by the use of sensors, body sensors and/or environmental sensors) coupled for example with machine learning can determine reliable assessments of situations. Big data translates into four main technical dimensions (volume, variety, velocity and veracity).

An EMG sensor can measure electrical signals produced by muscles during contractions or at rest. Nerve conduction studies are often done together with measuring the electrical activity in muscles, since nerves control the muscles in the body by electrical signals (impulses) and these impulses make the muscles react in specific ways. Nerve and muscle disorders can cause the muscles to react in abnormal ways, and be linked to a level of pain.

A pulse oximetry can measure oxygen saturation using a non-invasive probe. A small clip with a sensor can be attached to the person's finger, earlobe, or toe. The sensor gives off a light signal that passes through the skin. According to the light absorption of oxygenated hemoglobin and total hemoglobin in arterial blood, the measurement is expressed as a ratio of oxygenated hemoglobin to the total amount of hemoglobin.

Humidity and temperature sensors can be used for measuring the temperature of the human body and/or the humidity of the immediate environment around a person. An alarm signal can be issued if a certain number of changes are measured.

Imaging sensors (camera, video cameras, etc.) can be used, to estimate pain and mood. By computer vision, data can be estimated or extracted or inferred from data streams.

In an embodiment, the system comprises a lab-on-a-chip sensor. A sensor can comprise a DNA chip.

Contextual sensors can be sensors which can be present in the environment (RFID tags providing GPS information, nutritional values of meals, etc.) or worn by the patient. Some may also be implemented in the body of the user. These sensors can assess the current lighting conditions (night, dark, sunny, etc.), can probabilistically assess or classify the ambient audio level (restaurant, nightclub, working environment, sleeping room, outdoor activities assessed by the presence of wind sounds for example, indoor activities assessed by the presence of particular acoustic responses or audio signals such as music for example), can determine a geographical location such as a GPS sensor for example, can perform human face detection (the device can continuously monitor this parameter in order to provide an appropriate response upon the detection of the face of the user looking at the system for example), can evaluate the distance to the eye of the user-a user looking at the system. Some sensors can detect the breath of the user (when the user stands very close to the device, for example, during the night). The sensors mentioned above can be combined.

Sensors can be interoperable. One or more sensors can be interdependent, forming a dependency scheme. Some others can be identified as independent. A graph can allow detection of super nodes, i.e. active regulation entries.

Contextual and body sensors can be combined together.

Access to sensors can be remote, via APIs for example. The Body Area Network of sensors can be adaptive, reconfigurable depending on activated sensors. The BAN can comprise sound amplifiers, anemometers to quantify breathing, cameras, gyroscopes, etc.

The emotions of the patient, while sleeping or awake, can be estimated (in the voice signal if applicable, movements of the face, etc) and further remotely communicated. Eye-tracking i.e. movement of the eyes of the patient can be measured or estimated. Geolocation can be used, for example to trigger particular pain management rules. Gestures can be quantified, thanks to the use of one or more accelerometers. One or more microphones can be used (to estimate the patient distress if applicable). Selective microphones can be used. Ear buds can monitor heart rates.

Shapes (of sensors, for example antennas) can be complex. Shapes of the sensor and/or the radiation device can optimize data capture and/or dose delivery. Some shapes can be advantageously employed, for example, butterfly-shaped, round, square, or rectangular. Advantageous shapes (for one or more sensors or dose delivery devices e.g. antennas thereof) can comprise one or more of a dihedral angle or solid angle, a cube, a cuboid, a parallelepiped, a tetrahedron, a pyramid, a prism, an octahedron, a dodecahedron, an icosahedron, a cone, a cylinder, a sphere, a spheroid, an ellipsoid, a paraboloid, a hyperboloid. Other shapes are possible. A specific complex advantageous spiral can comprise one or more parts of an Archimedean spiral, Cornu spiral, Fermat's spiral, hyperbolic spiral, logarithmic spiral, Fibonacci Spiral (golden spiral) for example.

Patterns (for dose delivery) can be diverse, in space and/or time.

Such patterns can be used to determine the delivery of doses.

Patterns can be symmetrical or asymmetrical. Patterns can comprise one or more of a tree, a fractal structure (e.g. to increase contact surfaces), a spiral, a flow, a meander, a wave, a dunes, a bubble, foam, a crack, a spot or a stripe. Geometrical shapes can use convex polyhedron, geodesic domes etc. Patterns can comprise tessellations (patterns formed by repeating tiles all over a surface). Groups of tilings can include wax cells (such as those in honeycomb). Tiles can be overlapping. Patterns can use regularly repeating three-dimensional arrays (e.g. crystal structure, Bravais lattices for lattice systems in three-dimensional space). Crystal shapes can be cube-shaped crystals. Other forms include but are not limited to arrays, tilings, pavements, reticulate structures, etc. Textile patterns are also possible (e.g. end-on-end, pin stripes, rain pattern, toile, etc. Surfaces can comprise one or more of a minimal surface, a ruled surface, a non-orientable surface, a quadrics, a pseudospherical surfaces or an algebraic surface. Some patterns can be controllable (e.g. configurable at start or dynamically, evolve over time, etc).

Time patterns (e.g. evolution of patterns over time) can be one or more of regular, irregular, periodic, aperiodic, continuous or intermittent, or even opportunistic deliveries (e.g. leveraging a favorable time window, for example if the patient does not move). Patterns can comprise in particular pulses, trains of pulses, sinusoids, etc)

Sensors for example can be arranged in array, data fusion, in a grid, in one or more interconnected graphs.

With respect to sensors and/or actuators, MEMS can be used. Synthetic biology can be used. DIY biology can be used. DNA synthesis can be used. CRISPR (Clustered regularly-interspaced short palindromic repeats) technology can be used. For example, the sensitivity or even the chemistry of an analyte sensor can be personalized.

### User interfaces

Embodiments of the invention can comprise one or more user interfaces (Ul). The system according to the invention and/or components thereof can be provided with UI or I/O (input/output) interfaces, providing control endpoints.

The UI can be a graphical User Interface (U.I.), in 2D (display screen) and/or in 3D (e.g. augmented and/or virtual reality), with or without haptic input and/or output devices. The UI also can comprise or be performed by audio (sounds, music, etc.), vibrations, odors or others (nervous influx, electrical signal, etc.).

User interfaces (man-machine interfaces and/or man-man interfaces) can use voice commands or text-to-speech or speech-to-text steps or technologies.

A diversity of display devices can be used to restitute dose values, pain levels, trends or other information related to pain management. For example, a retinal laser display can be used. One or more pico-projectors can be used, for opportunistic display of information on surroundings surfaces in the environment.

A display may be integrated in head-mounted displays. A head-mounted display can be a display device, worn on the head, which can have a small display optic in front of one (monocular) or each eye (binocular). A typical head-mounted display can have either one or two small displays with lenses and semi-transparent minors embedded in a helmet, eyeglasses (also known as data glasses) or visor. The display units are miniaturized and may include CRT, LCDs, or OLED. Head-mounted displays can differ in whether they can display just a computer-generated image, show live images from the real world or a combination of both. Some head-mounted displays can allow a computer-generated image to be superimposed on a real-world view. This is sometimes referred to as augmented reality or mixed reality. Combining real-world view with computer generated image can be done by projecting the computer-generated image through a partially reflective mirror and viewing the real world directly. This method is often called "Optical See-Through". Combining real-world view with computer generated image can also be done electronically by accepting video from a camera and mixing it electronically with computer generated image. This method is often called "Video See-Through".

In a virtual retinal display, also known as a retinal scan display or retinal projector, a raster display (like a television) is generated directly onto the retina of the eye. The use of a coherent source (such as a laser diode) allows such a system to draw a diffraction limited spot on the retina. The light beam can be intensity modulated to match the intensity of the image being rendered. The user sees what appears to be a conventional display floating in space in front of them. Virtual retinal display system also can show an image in each eye with a very little angle difference for simulating three-dimensional scenes. Another important advantage can be privacy since only the intended user is able to see the image displayed.

Inputs devices can comprise devices such as one or more physical buttons, a touch screen or a portion thereof, a voice recognition device, eye-tracking device, etc. A wide range of haptic devices can also be used and such devices also include motion gestures analysis or interpretation. The devices can be combined with one another (multimodal interaction). For example, a voice command can be confirmed or modulated by an action on a touch sensitive interface.

Touch-sensitive surfaces can comprise sensors to detect intensity of contacts on the touch-sensitive surfaces. Such devices ("force touch") can use intensity thresholds or ranges of thresholds. Force touch can encode particular user interactions (speed of touch and force can confirm a bolus or even indicate hesitations or particular mood of a patient, since some biometry can be derived from user interactions, as keyboard typing). A touch imparted on the touch-sensitive display can cause a force sensor to undergo an electrical change in resistance that corresponds to a force imparted by the touch. The change in resistance may occur due to a change in geometry of the deflected or displaced material and the change in resistivity of the material arising from micro-changes in the structure of the material under pressure. Generally, between about 1 and 5 N of force may be applied by a user to the touch-sensitive display. Force sensor(s) can be force sensitive resistors, strain gauges, strain sensors, piezoelectric or piezo resistive devices, pressure sensors, or other suitable devices. Various patterns of the force sensors can be used, such as patterns of a single, continuous sensor or patterns of multiple discrete sensors electrically coupled to one another or in isolation. Other patterns, such as multiple force sensor patterns, e.g., bi-directional, multi-grid patterns, may provide increased sensing accuracy with less dependency on the width and orientation of the pattern or the direction of the touch. For example, planar or stacked rosette patterns, such as "tee", "delta," and "rectangular" rosettes, may be utilized. Force can refer to force measurements, estimates, and/or calculations, such as pressure, deformation, stress, strain, force density, force-area relationships, thrust, torque, and other effects that include force or related quantities. In some embodiments, the scroll speed or the quantity of data selected (or other logic, with medical significance) can be adjusted in response to the magnitude of force. A gesture can be characterized by, but is not limited to a pinching, sliding, swiping, rotating, flexing, dragging, or tapping motion between or with any other finger or fingers. A single gesture can be performed with one or more hands, by one or more users, or any combination thereof.

A triggering information can be automatically provided by a sensor, wherein the sensor, is selected from a group, or is a combination thereof, comprising: a sensor adapted to perform human face detection, a sensor adapted to evaluate the distance to an human eye, a sensor adapted to detect or analyze breath or smell, a sensor adapted to interpret acceleration data or movements, a sensor adapted to monitor environmental or contextual conditions such as lighting conditions, a sensor adapted do determine an ambient audio level, a sensor adapted to determine a geographic location such as a GPS device, an electroencephalography EEG sensor, an electrocardiography ECG sensor, an electromyography EMG sensor, a sensor adapted do determine a body temperature, a sensor adapted to monitor continuously or discretely a user body parameter such as a blood glucose level or a heartbeat rate or a blood cholesterol level or a blood alcohol level or a blood analyte level. For example, a camera incorporated on the medical device can estimate the mood of the user, or the distance to his face, or estimate the field of vision and provide appropriate responses. In another example, if the accelerometer history indicates that the patient is confused (number of accelerations recorded by the accelerometer above a certain predefined threshold for example), and/or in hypoglycemia state (which may cause the vision to be troubled) the system can display some predefined specific data. This automatic mode can be enabled by a cooperation of sensors.

Display, user input and data model can be intermingled or combined. Relationships between these three abstractions can be associated with concepts such as influence, feedback, ponderation, limitation, activation, deactivation, control or command. For example, the data priority scheme can influence or drive or control the display logic. For example, if a pain event probability or event is determined, associated alerts or values can preempt or replace any other display data. User interactivity and machine behavior can be defined by user-defined preferences or by machine learning or driven by rules retrieved from the network.

In some embodiments, the administration of dose can be remotely controlled and optionally can be assisted with haptic devices, so that the dose administrator can have realistic feedbacks.

In an embodiment, the user of the dose delivery device can define the delivery speed by moving a finger on a touch screen, thus determining the dose delivery which can be performed in real-time, as the gesture is executed. In some embodiments, the dose delivery is postponed in time. In some embodiments, dose profiles are sketched on a touch screen (e.g. with rescaling options).

A diversity of displays can be used. Regarding displays, augmented reality can be used (e.g. a projector or pico-projector can display dose values on the wall or ceiling). Holographic displays can be used. Electronic Braille displays can be used. Touch screens can be used. Display can use force feedback or haptic mechanisms.

Brain machine interfaces can be used. One or more displays can be placed on the module and/or on the remote controller and/or on the smartphone or smart watch associated with the system according to the invention. Displays embedded in smart glasses and/or a smartphone and/or a smart watch can be used. Projectors can be used. A display can comprise one or more of an electronic ink screen or a touch screen or a Braille screen or an OLED screen (or a combination thereof). Opportunistic display can be used (available devices in the vicinity of the system can be accessed and caused to display one or more pain levels and/or dose values and/or warnings (in case pain levels exceeding predefined thresholds). Screenless computing systems also can be used (holograms, virtual retinal display or Retinal Direct, and Synaptic Interface such as Braille or sending signals from electronic devices such as cameras into brains or certain neurons).

A diversity of gestures can be used to enrich the interaction with pain management system. Information can scroll. Slide-to-refresh, slide-to-unlock gestures can be used. Force feedback can be used.

For the user experience and the man-machine interaction, a wide diversity of technologies can be used. Speech synthesis can be used (e.g. to enunciate BG values or trends). Text-to-speech can be used (e.g. upon request). Imaging sensors combined with OCR capabilities embedded in software apps can allow the user to acquire an image of the food packaging and automatically extract carbs value for the amount having being eaten. Accelerometers or machine vision can allow to estimate the numbers of swallowing by the patient and to further correlate with carbs intake.

Comprehensibility can be improved for example by using pictograms or audio-guided instead of merely textual information.

Virtual reality and/or augmented reality interfaces can be used to manage pain and/or handle the systems according to the invention. User interfaces can be used for the display of information and/or interactivity with the user (e.g. reception of inputs or selections). For example, one or more graphical overlays can be used to indicate the blood glucose value (i.e. graphical elements can be superimposed to the field of view of the user). In an embodiment, the color of the sky can be changed (from dark blue for low values to red for high values; similar or equivalent gradients). In an embodiment, an instant blood glucose value can be opportunistically displayed onto one or more objects in the environment (e.g. windshield). The display can be effective (i.e. using a projector) or can be virtual (e.g. head-mounted display, with semi-transparent glasses, retinal display, etc.). Display can combine virtual display and real display (for privacy purposes). For example, a red circle can be projected onto a table while the actual value is displayed within said "real" circle. Haptic feedbacks also can be used, for example in combination with displays (for example with progressive intensity). Using virtual and/or augmented reality advantageously can reveal to be non-intrusive and progressively warn the user about instant measures and/or trends (seamless integration, as natural as possible). Via user preferences, notifications can be personalized (for example by defining preferred spatial locations for notifications, depending on types of data, using geofencing rules, etc.). For example, a user may prefer notifications to de displayed up in the sky, or down on the floor if walking in the street, etc. Another user may prefer a special part of the body (e.g. the right hand or a specific finger) to be the preferred location place for notifications. Notifications may float up in the air, be displayed on available hardware screens (picture-in-picture), be centralized with smartphone notifications or to the opposite be separated from it. Snooze and reminders options can be setup with voice commands, gestures, or a combination thereof.

Augmented reality and/or virtual reality can be advantageous for pain management. Augmented reality (or "mixed reality") can allow creating a fictional layer on top of the real-world context. Said layer can be generated depending on user and/or context data. Virtual media (text, documents, multimedia) can be triggered by location, for example to create a fictional set of events occurring in the real-world space. Place-based augmented reality games can be played in specific real-world locations. User experience can be enriched with additional data (text, numerical data, audio, and video). An event in pain management, even if properly handled, can be further enriched by using one or more associated simulations, so that the user can learn better and faster. Associated past errors (e.g. insulin stacking) can be reminded to the user and further contextualized. Because a given therapy just occurred, the user can be more receptive to learn further lessons and/or advices. A pet or animal can be simulated for. learning purposes (children can learn pain management in a softly manner).

Wearable computer can execute software or apps. Wearable computers (e.g. computerized sensors) advantageously can improve pain therapy. A pain management app for example can display pain timecards to the user. Users may view (and possibly share) their timecards on-demand or according to configurable notifications.

Another use of AR/VR can be to provide users with interactive and "how to" guides (manipulating the emitter, charging). For example, the various gestures can be (subjectively) displayed in overlay, step by step, in context so that the user is optimally assisted in the therapy (following the subjective view or a contrario from different angles, at different playback speed, etc)
AR/VR devices can optionally include one or more haptic devices or components, operable to provide a tactile sensation to a user. For example, a haptic device can provide a tactile sensation of pressure and/or texture when touching virtual content (e.g., virtual objects, virtual tools, other virtual constructs). The tactile sensation can replicate a feel of a physical object which a virtual object represents. In some embodiments, haptic devices can be worn by the user (user wearable glove, haptic totems, etc.). One or more devices can detect and interpret user gestures into commands.

The system according to the invention can comprise a brain-computer interface (BCI) or mind-machine interface (MMI) or direct neural interface (DNI) or brain-machine interface (BMI). Such expressions designate a direct communication pathway between an enhanced or wired brain and an external device. A brain-computer interface encompasses any form of controlling a computer via a direct electrical connection to the human body. The user also can control the various user interfaces described herein (in particular any one of the AR/VR embodiments). BCIs can be invasive or not, EEG based or non-EEG-based (e.g. pupil-size oscillation). BCIs generally use a combination of EEG (electroencephalography), EMG (electromyography), EKG (electrocardiography), and accelerometer data. BCIs and eye-tracking can be combined.

Display devices can cooperate (display can be distributed). One main screen or display may handle the display of all or part of the medical data, but several displays may handle in cooperation the "global" display (i.e. the interaction towards the user).

### Association Schemes

In some embodiments, parts of the system according to the invention can be arranged and/or configured according to association schemes.

Subparts of the system can be (e.g. physically) arranged and/or (e.g. logically) configured (or adapted) according to different schemes. To get a robust combination, one or more components can be redundant (duplicated or triplicated). The components of the system can be distributed (e.g. "body area network") and/or centralized. The association between the one and more emitters and/or the one or more sensors can be performed in different ways. Association can reversible (e.g. releasable) or irreversible. Association can one or more of adhesive e.g. Gecko-based adhesive, aerogel, glue, Velcro, magnetic (releasable), electrical, pressure-based, etc. The one and more sensors and/or the one or more sensors and/or the one or more dose delivery actuators can be located adjacent from another, or at remote distance (body area network, cloud, etc).

Association or pairing between apps can use QR codes, or barcodes, or tokens, or communication protocols, with or without the intervention of a user. The disconnection of one or more parts can trigger an alarm.

The extension can be integrated or inserted or melted within an e-textile, or use flexible electronics. The extension can be 3D printed. It can be integrated in textile.

Regarding hardware, flexible wired connections can be used. FPGA circuits can be used to provide faster responses times and higher resistance to cyber-attacks. The architecture of the system can be fractal. Cloud computing resources can be used (or "grid").

### Communication Schemes

Data communication can use fiber optics and/or lasers. In an embodiment, quantum key distribution can be used for the different parts of the architecture to define and share one or more secret keys, the further classical encryption of further data exchanges using said keys. In an embodiment, post-quantum cryptography can be used.

In order to avoid interception or eavesdropping, data can be streamed (i.e. no complete data can be captured at a given moment), at least in parts.

Cognitive radio technology, also known as smart radio can allow different radio technologies to share the same spectrum efficiently by adaptively finding unused spectrum and adapting the transmission scheme to the requirements of the technologies currently sharing the spectrum. This dynamic radio resource management is achieved in a distributed fashion and relies on software-defined radio.

A cognitive radio (CR) is an intelligent radio that can be programmed and configured dynamically. Its transceiver is designed to use the best wireless channels in its vicinity. Such a radio automatically detects available channels in wireless spectrum, then accordingly changes its transmission or reception parameters to allow more concurrent wireless communications in a given spectrum band at one location. This process is a form of dynamic spectrum management.

Li-Fi technology can be used. Li-Fi can facilitate high-speed data transmission via pulsating light sources.

Communications can use any of a plurality of communications standards, protocols and technologies, including but not limited to Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (Wi-Fi) (e.g., IEEE 802.11a, IEEE 802.11ac, IEEE 802.11ax, IEEE 802.11b, IEEE 802.11g and/or IEEE 802.11n), voice over Internet Protocol (VoIP), Wi-MAX, a protocol for e-mail (e.g., Internet message access protocol (IMAP) and/or post office protocol (POP)), instant messaging (e.g., extensible messaging and presence protocol (XMPP), Session Initiation Protocol for Instant Messaging and Presence Leveraging Extensions (SIMPLE), Instant Messaging and Presence Service (IMPS)), and/or Short Message Service (SMS).

### Security Schemes

In some embodiments, the system or parts thereof are arranged and/or configured according to one or more security schemes.

Security schemes can comprise a Physically Unclonable Function and/or a challenge-response test and/or a True Random Number Generator.

Various mechanisms can be used to improve security and/or safety of dose delivery according to the invention.

Communications can be encrypted and/or obfuscated. Security of the system according to the invention or of specific part thereof can be protected using one or more of the technologies or mechanisms comprising asymmetrical encryption like AES, public key encryption like PGP or GPG, physically unclonable function or PUF, crypto ledger or blockchain, proofs-of-work, quantum key distribution, post-quantum cryptography, etc. Also steganography can be used (e.g. pain management reports can be concealed a file, message, image, video or within another file, possibly of no particular subjective interest).

Biometrics can be used to grant access to the system. End-to-End encryption can be used. Token-Based Access Control can be used.

Cyber-attacks against the system can be prevented by the use of Turing tests (e.g. challenge-response for bolus administration after a value is buffered but not actually injected, etc.). In an embodiment, the extension according to the invention can serve as a gateway for security purposes. For example, the extension can implement or participate to a Turing challenge (e.g. a CAPTCHA), ensuring that a human being is forming request to retrieve a dose value (radiation dose which can benefit from such a testing scheme). The extension also can embed one or more ciphering keys, which can be required for a global chain of devices to properly work (Digital Rights Management) or to be authorized to function.

"Secure boot" or "verified boot" can be used. One or more described subsystems according to the invention can use secure and/or verified boot. One or more devices of the system can be secured, by a "secured boot" or a "verified boot" (for example, hash values at startup can be compared with authorized values). Such embodiments advantageously can defeat sabotage or cyber-attacks. If not successfully verified, a safety-critical device can be executed in a downgraded state (e.g. specific functions can be forbidden for execution)

Hard switch or hard-off switch can be used (for example to deactivate enhanced mode of pain management, or particular rules, which may reveal to dysfunction).

Wired communication can be required to avoid eavesdropping or attacks (such as man-in-the-middle attacks). While wireless communications are generally efficient, it may reveal advantageous to require wired connections.

Onion-routing or TOR networks can be used. Onion-routing can be combined by techniques for preserving anonymity (e.g. proxies, P3P, etc.).

Communication of data can use the BitTorrent protocol, for example combined with TOR and/or zero-knowledge mechanisms.

Some hardware can be triplicated. Triple modular redundancy (TMR) is a fault-tolerant form of N-modular redundancy, in which three systems perform a process and that result is processed by a majority-voting system to produce a single output. If any one of the three systems fails, the other two systems can correct and mask the fault. TMR can be used for different parts of the invention. Triple modular redundancy hardware can be faster than Hamming error correction software.

Computer security of hardware and/or software embodiments can be improved using mechanisms comprising formal verification of code (e.g. automated theorem proving), two-factor authentication, regular security patches and updates, use of a security scanner, automated audit trails, dongles, trusted platform modules, intrusion-aware cases, drive locks, disabled USB ports, use Virtual Private Networks (VPNs), computer case intrusion detection (e.g. push-button switch), encrypt hard drives, biometric validation (such as thumb print readers), use of secure coding techniques, access control lists, interference shields, etc. Trusted computing techniques can be used (e.g. using one or more of an endorsement key, secure input and output, memory curtaining/protected execution, sealed storage, remote attestation, etc).

In an advantageous embodiment, a fingerprint sensor is associated with the emitter device. In order to be validated (e.g. reimbursement schemes) or otherwise legitimated, there can be required to authenticate by biometrics, for example when uploading data for analysis by the backend (e.g. machine learning).

In some embodiments, one or more parts of the invention can be optionally secured with a physically unclonable function (PUF). A PUF is a physical structure which is generally easy to evaluate but hard to predict. An individual PUF device is generally impossible to duplicate, even given the exact manufacturing process that produced it. In this respect it is the hardware analog of a one-way function (e.g. a challenge-response). A PUF can be used for key generation (enabling authentication for example). A PUF can provide a collection of responses with predefined ranges of values and properties (randomness, aging, entropy, etc). Using one or more PUFs in devices is advantageous.

In some critical embodiments, Quantum Key Distribution (QKD) can be used. Quantum key distribution can be enabled on mobile devices. QKD is used to produce and distribute a key, not to transmit any message data. The key can then be used with any encryption algorithm, such as AES. In some embodiments, a pseudo random number generator can be used. In some embodiments, a quantum random number generator can be used.

### Cryptographic schemes

In some embodiments, the system according to the invention, or parts thereof, can be arranged and/or configured according to one or more cryptographic schemes.

Cryptographic schemes comprise a Quantum Key Distribution mechanism and/or post-quantum cryptography and/or quantum-safe cryptography and/or crypto-ledger and/or one or more smart contracts configured to control or influence operations of the device and/or communications thereof. Public keys schemes and/or symmetrical encryption can be implemented.

Security of networks of sensors (e.g. Internet of Things) can be performed in several manners, for example by using one or more symmetric keys with gateways, by selectively protecting vital and immutable packet parts with message authentication code(s) with encryption, or by using message authentication codes. Other mechanisms include one or more of puzzle-based defense mechanisms, ad-hoc security domains, chains of certificates, privacy aware identifiers used to prevent unauthorized user tracking, built-in mobility signaling or combinations thereof.

### Pain management Rules

In some embodiments, the system, parts thereof and/or the control thereof can be arranged and/or configured according to one or more pain management rules.

Pain management rules can be specific to/for particular medical conditions, for example for pain management. Some rules can be FDA-regulated. Some others may not be (private use).

Advantageously, the implementation of rules can place the patient at the heart of pain management (personalized system), can provide algorithms as "tools", among other "tools" (place them as concurrent "offers"), can set transparency at all levels (hardware specifications, Open-Source Software, algorithms assumptions and models). Open or free software can lead to faster development, to a kind of "immortal" code (i.e. fork-able code base).

In some embodiments, the metabolism of the patient can be measured and/or estimated and/or simulated and/or computed, directly and/or indirectly, statically and/or dynamically.

Pain management rules can use one or more metrics. Anonymized data can be aggregated and sensors analytics can be public. Botnets (collection of computers) can data-mine the aggregated data. The rules can be configurable in the Cloud by the patient. In some embodiments, the patient can configure time intervals and/or thresholds for notifications.

Rules can handle the handling of exceptions by general amplifiers or attenuators, e.g. "increase all thresholds applicable to dose by 20% if corporal temperature has exceeded more than 40° C. during 2 hours over the last past 12 hours". Rules can handle specific triggers. Rules can handle reminders.

In an embodiment, rules are expressed in natural language by the patient and/or parent and further converted into formal logical rules. In an embodiment, fuzzy logic is used.

Heuristics (machine-readable) and/or rules (human-readable) can be implemented on request in the system according to the invention.

Rules can be ordered hierarchically.

In an embodiment, a plurality of logical or software rules can govern the hardware according to the invention. The personalization or configuration of pain management rules and the further assembly or combination of such rules can lead to a DIY Do-it-Yourself system. In an embodiment, each rule can be associated with a FDA score, each combination can be associated with a specific score (e.g. in terms of reliability, performance, systemic risks assessments, etc). Particular combinations may be forbidden. Some other rules may be recommended. Rules or combinations of rules ("packages") can be downloaded and further installed. Rules can be protected by DRM. Some can be open-sourced, while some others can remain in binary forms. Some can be insured, some others not.

The correlations or covariance or invariance or coupling of sensors (by pairs or more) can be determined, in many different independent or combined ways. Software agents can crawl the web corpus to establish correlations and patterns, the identification of critical parameters, specific to an individual. Human crowd sourcing also can browse and back-test data to identify composite data combinations improving hypo detections. Social networks can be used (e.g. estimation of carbohydrates values of images of meals).

The physiology of the patient can be modeled, for example with deep learning. A virtual clone of the patient can enable to test injections and estimate future BG values or trends.

The User Interface to define or use or configure rules can use gamification. Head-mounted displays can be used (or "glasses" or virtual reality helmets).

### Pain management

Pain management, by dose deliveries by the described system, can be handled in various ways.

Interfaces can allow the patient to handle, visualize and configure personalized rules for pain management.

The rules can be configured in an interactive system. One or more intermediaries can handle, filter and enhance the data at each step of the algorithmic chain.

A rule can be location-based. A rule can be locked, conditionally authorized (depending on the context, requiring payment, etc). A rule can be free, require payment or can be installed for free with advanced features and/or settings requiring a payment.

Machine-to-machine communications can occur, for example between modeled physiologies (set of rules 1 made for patient profile 1 can be tested with a profile 2).

The rules for pain management can be personalized. Personalized rules can be scored by comparison with FDA approved pain management rules. Approved pain management rules can take into account systemic risks (i.e. specific combinations of sensing and delivery devices which could not allow patients to take appropriate measures).

Pain management rules ("rules") can be use priority mechanisms (a rule can be associated with a priority and a plurality of competing rules can be executed in parallel, a selection and/or coordination among rules results or predictions can be performed). Pain management rules can preserve the patient's privacy. Rules can be public (standardized rules, with no configuration data for example) or private (specifics can be confidential, for example the number of boluses, so that to avoid excessive surveillance attempts by insurance companies for example). Pain management rules can be probabilistic. Pain management rules can be programmable, in part or in full. Pain management rules can be advertised and/or ranked. Via social networks, patients can rate, score or comment one or more rules or recommendations so that to improve learning curves and/or suggest rules' improvements (for example). Pain management rules can be simulated (installed in a sandbox, to estimate resulting BG values, knowing the lifestyle and past BG values of a patient). Search engines can index and rank by relevancy available pain management rules according to the user profile. Pain management rules can include structured testing, reminders or alarms, bolus or basal injection patterns or complex rules based on sensor's data (heart rate, audio level, wetness, vibrations etc).

Pain management rules can be scripted. In procedural knowledge, scripts are like frames, except the values that fill the slots must be ordered. A script can be a structured representation describing a stereotyped sequence of events in a particular context. Scripts can be used in natural language understanding systems to organize a knowledge base in terms of the situations and conceptual transitions that the system should understand.

Pain management rules can comprise or use smart contracts. Smart contracts comprise computer protocols which facilitate, verify, or enforce the negotiation or performance of a contract (or that make a contractual clause unnecessary). Contractual clauses can be made partially or fully self-executing and/or self-enforcing, reducing transaction costs associated with contracting. The provision of tangible devices and/or software rules can be regulated with the use of such smart contracts. In an embodiment, physical objects can be micro-tagged with contractual requirements (e.g. payment can be conditional or enforced for certain types of uses of certain predefined types of information).

In an embodiment, users can subscribe to one or more "channels" (e.g. trustable persons or corporation entities) delivering or proposing pain management rules.

The social graph of users of pain management rules can be analyzed. Super-nodes can be identified (e.g. users with intense social activity, trusted users, influencers, etc).

"Pain management" according to the invention for example designate the evaluation of carbs of a meal given one or more pictures thereof, determination of bolus and/or basal value, analysis of trends and predictions based on raw data, or more generally any therapeutic measure, determination, action or evaluation.

Pain management according to the invention can use involve various data sources, e.g. human mechanisms and/or machine technologies. For example, pain management can use one or more of data mining, deep learning, beam search, LDPC-codes, neural network, etc.

Pain management according to the invention can use "machine learning", e.g. supervised learning, for example by identifying of patterns in BG values (e.g. postprandial profiles, exercise profiles, at night, etc).

Pain management according to the invention can use deep-learning (this field for example comprises one or more of techniques comprising sparse coding, compressed sensing, connectionism, reservoir computing, liquid state machine, echo state network, supervised learning, classification, regression, clustering, dimensionality reduction, structured prediction, anomaly detection, neural nets, machine learning venues, artificial neural networks, deep neural network architectures, back propagation, convolutional neural networks, neural history compressor, recursive neural networks, long short term memory, deep belief networks, convolutional deep belief networks, deep Boltzmann machines, stacked (de-noising) auto-encoders, deep stacking networks, tensor deep stacking networks, spike-and-slab RBMs, compound hierarchical-deep models, deep coding networks, deep q-networks, networks with separate memory structures, LSTM-related differentiable memory structures, semantic hashing, neural Turing machines, memory networks, pointer networks, encoder-decoder networks, multilayer kernel machine, etc).

Pain management according to the invention can use web services. A Web service is a service offered by an electronic device to another electronic device (machine-to-machine communication), communicating with each other for example via the World Wide Web. Major classes of Web services comprise REST-compliant Web services (manipulation of representations of Web resources using a uniform set of stateless operations) and Arbitrary Web services (in which the service may expose an arbitrary set of operations). In particular, a Web API is a development in Web services with a simpler representational state transfer (REST) based communications. RESTful APIs do not require XML-based Web service protocols (SOAP and WSDL) to support their interfaces.

Pain management according to the invention can use web services service-oriented architecture (SOA). SOA is an architectural pattern in computer software design in which application components provide services to other components via a communications protocol, typically over a network. SOA generally encapsulates application logic in services with a uniformly defined interface and makes these publicly available via discovery mechanisms.

Pain management according to the invention can use so-called web 2.0, mashups of applications or APIs. Web 2.0 designates the ability of visitors to contribute information for collaboration and sharing. Web 2.0 applications generally use RESTful web APIs and AJAX based user interfaces, utilizing web syndication, blogs, and wikis. Pain management according to the invention can use service-oriented business applications (SOBAs).

Pain management according to the invention can use technologies of the "Internet of Services", wherein people, machines, and goods have access via the network infrastructure. Micro services can be used (interpretation of service-oriented architectures used to build distributed software systems, by using technology agnostic protocols).

Pain management according to the invention, for example pain management rules, can use various time mechanisms e.g. time-to-live (TTL), timers, etc.

Pain management according to the invention can use Error-correction code ECC or Forward error correction FCC (this field for example refers to or comprises one or more of techniques comprising concatenated FEC codes for improved performance, low-density parity-check LDPC, turbo codes, etc).

Pain management according to the invention can use turbo codes or turbo codes (one or more of AN codes, BCH code, Berger code, Constant-weight code, Convolutional code, Expander codes, Group codes, Golay code, Goppa code, Hadamard code, Hagelbarger code, Hamming code, Latin square based code for non-white noise, Lexicographic code, Long code, Low-density parity-check code, also known as Gallager code, LT code, Fountain code, online code, raptor code, reed-Solomon error correction, reed-Muller code, repeat-accumulate code, repetition codes such as Triple modular redundancy Spinal code, Tornado code, Walsh-Hadamard code, Viterbi algorithm, Soft-decision decoding, Interleaver BCJR algorithm, serial concatenated convolutional codes, turbo equalizer

Pain management according to the invention can use fuzzy-logic (natural language interfaces, e.g. rules expressed in a way which is easy to understand and/or modify by the user and which is manipulatable by the computer). This field for example refers to or comprises one or more of techniques comprising adaptive neuro fuzzy inference system ANFIS, artificial neural network, defuzzification, expert system, false dilemma, fuzzy architectural spatial analysis, fuzzy classification, fuzzy concept, fuzzy Control Language, fuzzy control system, fuzzy electronics, fuzzy subalgebra, fuzzyCLIPS, High Performance Fuzzy Computing, IEEE Transactions on Fuzzy Systems, Interval finite element, Neuro-fuzzy techniques, noise-based logic, rough set, sorites paradox, type-2 fuzzy sets and systems, vector logic)

Pain management according to the invention can use Bayesian inference (this field for example refers to or comprises one or more of techniques comprising admissible decision rule, Bayesian efficiency, Bayesian probability, Probability interpretations, Bayes' theorem, Bayes' rule, Bayes factor, Bayesian network, Prior Posterior, Likelihood Conjugate, prior, Posterior, predictive, Hyperparameter, Hyperprior, Principle of indifference, Principle of maximum entropy, Empirical Bayes method, Cromwell's rule, Bernstein-von Mises theorem, Bayesian information criterion, Credible interval, Maximum a posteriori estimation, Bayesian linear regression, Bayesian estimator, Approximate Bayesian computation, Bayesian hierarchical modeling, Bayesian Structural Time Series, Monty Hall problem

Pain management according to the invention can use LDPC-codes (this field for example refers to or comprises one or more of techniques comprising belief propagation, graph theory, Hamming code, linear code, sparse graph code, expander code).

Pain management according to the invention can use other capacity-approaching codes (e.g. comprising serial concatenated convolutional codes, online codes, fountain codes, raptor codes, repeat-accumulate codes, Tornado codes or Polar codes).

Pain management according to the invention can comprise one or more pain management rules. Algorithms associated with pain management rules can be executed locally, i.e. on a computing device in the vicinity of the user. Alternatively or as a complement (elastic computing), remote computing resources can be used.

Privacy-techniques can be used. A range of techniques can be combined with embodiments of the invention. Various techniques can be used, possibly in combination. Some of these techniques or steps are described hereinafter.

"Homomorphic encryption" can be used. Homomorphic encryption is a form of encryption that allows computations to be carried out on ciphertext, thus generating an encrypted result which, when decrypted, matches the result of operations performed on the plaintext. Such use advantageously enables to preserve privacy.

"Secure multi-party computation" can be used. SMPC is a subfield of cryptography enabling the parties to jointly compute a function over their inputs while keeping those inputs private

"Virtual Party Protocol" can use virtual parties and mathematics to hide the identity of the parties.

"Secure sum protocols" can be used to allow multiple cooperating parties to compute sum function of their individual data without revealing the data to one another

"Differential privacy" can be used. DP is a technique for releasing statistical information about a database without revealing information about its individual entries. DP can maximize the accuracy of queries from statistical databases while minimizing the chances of identifying its records.

"Quasi-identifiers" can be used. When combined, QI become personally identifying information.

"Exponential Mechanisms" can be used. With EM, one can output a synthetic dataset in a differentially private manner and can use the dataset to answer queries with good accuracy." "K-anonymity" can be used. Given person-specific field-structured data, produce a release of the data with scientific guarantees that the individuals who are the subjects of the data cannot be re-identified while the data remain practically useful.

Pain management and/or algorithms can use can comprise interpolation steps, iterative, recursive steps.

Pain management can use feed-forward mechanisms (with or without feedback mechanism). These mechanisms can relate to control theory, physiology/biology or computing and can prove advantageous for pain management. Feed-forward designates an element or pathway within a control system which passes a controlling signal from its external environment to a load elsewhere in its external environment. A feed-forward system responds to its control signal in a pre-defined way without responding to how the load reacts. In contrast, a system with a feedback mechanism adjusts the output to take account of how it affects the load (the load itself can vary unpredictably, and the load is generally considered to belong to the external environment of the system). In a feed-forward system, the control variable adjustment is not error-based: it is based on knowledge (e.g. in the form of a mathematical model) of the process and knowledge about or measurements of the process disturbances. Pure feed-forward control without feedback can be called "ballistic", because once a control signal has been sent, it cannot be further adjusted (any corrective adjustment must be by way of a new control signal). By contrast, "cruise control" adjusts the output in response to the load that it encounters, by a feedback mechanism.

Algorithms for pain management (e.g. evaluation of carbs of a meal given a picture thereof, determination of bolus value, etc) can involve various sources and/or technologies, comprising crowd sourcing and social networks, or human evaluation (e.g. by doctors) along with machine algorithms. Advices (e.g. proposals of rules and/or dose values or profiles) can be taken into account, by "pull" (e.g. upon request by the patient, for example in an uncertain situation or in a hurry) and/or "push" (for example, different opinions are collected and ranked, for later display to the patient).

### Social Mechanisms

In some embodiments, the system, parts thereof and/or the control thereof can be arranged and/or configured according to one or more social mechanisms.

A diversity of social features can be used. Crowd of users can discuss pain level values and/or trends, and comment on tips and tricks to handle pain. Real-time encrypted chats among peers can encourage dialog and improve therapy or adherence to therapy.

Usage data can be gathered and anonymized. Statistics can be derived from this collection. Homomorphic encryption can be used (logical operations performed on encrypted data).

Some systems according to the invention can be operated in so-called "stealth" mode or "camouflaged" mode. For example, pain management app can be disguised into a classical software app and the reference to pain can be obfuscated.

### Time and/or Space Schemes

In some embodiments, the system, parts thereof and/or the control thereof are arranged and/or configured according to one or more time and/or space schemes (for measurements and/or dose deliveries).

Dimensions of sensors and/or actuators can be different (e.g. macro or micro-scales).

Structured testing can comprise different schedules to retrieve data.

Measurements and/or radiation dose deliveries can be performed "continuously" and/or "continually" and/or ""intermittently" (regularly or irregularly) performed.

Doses can be regular or irregular, periodic or a periodic, intermittent, opportunistic (triggered by predefined event, available bandwidth, etc).

Doses can be logged. History of logs can be archived. Logs can be encrypted.

Does can be event-driven (e.g. movement while sleeping)

Time management is an important factor in pain management. Various time management can be implemented, for example using one or more of a timeout, a timer, a timestamp:

Time can be divided in hours minutes and seconds but specific pain time units can be defined, for example in relation with residual insulin. Graphical indicators can be implemented (remaining time, residual insulin, time before hypoglycemia, etc). Specific custom clock faces can be determined for pain. Adherence to therapy can be encouraged by adapted user interfaces (e.g. indicating progress, marking rewards, providing warnings, etc).

Some embodiments of the invention can be achieved at different sizes or scales or size scales. Some components or parts or portions can be miniaturized. Dimensions can be macroscopic (as it is generally the case today), millimetric, microscopic, sub-microscopic if not at nanoscale.

### Radiation delivery triggers

Dose delivery can be managed in various ways. Factors or parameters taken into account for radiation delivery can depend on multiple values measured and/or computed. The nature of these parameters can comprise user declared data (e.g. declarations of mood, pain feeling) and/or user measured data (e.g. biomarkers' presence, heart rate, perspiration, etc), and/or environmental data (e.g. time of day, schedule of the week, agenda, pollution level for ease of respiration, etc).

Various "situations" and/or "states" can be defined from such data. Situations for example can be classified into "calm", "busy", "hectic", "frantic", with associated quantified nuances. States can comprise psychological or otherwise emotional predefined states (e.g. "focused", "quiet", "anxious", "resting", "working", "happy", "depressive", etc). States and situations can be weighed or otherwise quantized.

For example, it can be predicted that physical activity can be reduced after lunch for a certain patient, remaining quiet or still, which situation can be favorable for a radiation dose. A *contrario*, it can be estimated that associated hyperglycemia following meal can inhibit pain feeling.

### Analytes and/or Biomarkers

In some embodiments, at least one sensor can determine the concentration of an analyte and/or of a biomarker. Depending on these values, dose delivery can be adjusted. Embodiments of the invention are applicable are applicable to humans and more generally to mammals (hosts).

A sensor associated to the emitter can be a sensor capable of determining the level of any suitable analyte in the body, for example, oxygen, lactase, insulin, hormones, cholesterol, medicaments, viruses, or the like.

Blood analyte or sample can be taken from capillary or interstitial or venous or arterial blood. A diversity of blood analyte can be measured. The one or more analyte being measured can comprise one or more of a substance in a biological fluid, a chemical constituent in a biological fluid, a substance or chemical constituent in a biological fluid that can be analyzed, a substance in blood, a substance in interstitial fluid, a substance in lymph fluid, a substance in urine, an artificial substance, a metabolite, a reaction product.

An analyte can comprise acarboxyprothrombin, acylcarnitine, adenine phosphoribosyl transferase, adenosine deaminase, albumin, alpha-fetoprotein, amino acid profiles, arginine, histidineurocanic acid, homocysteine, phenylalaninetyrosine, tryptophan, andrenostenedione, antipyrine, arabinitol enantiomers, arginase, benzoylecgonine (cocaine), biotinidase, biopterin, c-reactive protein, carnitine, carnosinase, CD4, ceruloplasmin, chenodeoxycholic acid, chloroquine, cholesterol, cholinesterase, conjugated 1-.beta, hydroxy-cholic acid, cortisol, creatine kinase, creatine kinase MM isoenzyme, cyclosporin A, d-penicillamine, de-ethylchloroquine, dehydroepiandrosterone sulfate, DNA, acetylator polymorphism, alcohol dehydrogenase, alpha 1-antitrypsin, cystic fibrosis, DuchenneBecker muscular dystrophy, analyte-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, 21-deoxycortisol, desbutylhalofantrine, dihydropteridine reductase, diptheriatetanus antitoxin, erythrocyte arginase, erythrocyte protoporphyrin, esterase D, fatty acidsacylglycines, free .beta.-human chorionic gonadotropin, free erythrocyte porphyrin, free thyroxine (FT4), free tri-iodothyronine (FT3), fumarylacetoacetase, galactosegal-1-phosphate, galactose-1-phosphate uridyltransferase, gentamicin, analyte-6-phosphate dehydrogenase, glutathione, glutathione perioxidase, glycocholic acid, glycosylated hemoglobin, halofantrine, hemoglobin variants, hexosaminidase A, human erythrocyte carbonic anhydrase I, 17-alpha-hydroxyprogesterone, hypoxanthine phosphoribosyl transferase, immunoreactive trypsin, lactate, lead, lipoproteins ((a), BA-1, .beta.), lysozyme, mefloquine, netilmicin, phenobarbitone, phenytoin, phytanicpristanic acid, progesterone, prolactin, prolidase, purine nucleoside phosphorylase, quinine, reverse tri-iodothyronine (rT3), selenium, serum pancreatic lipase, sissomicin, somatomedin C, etc.

An analyte also can comprise one or more of a metabolic product, an hormone, an antigen, an antibody and/or one or more trace elements (e.g. adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measlesmumpsrubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzirangeli, vesicular stomatis virus, Wuchereria bancrofti, yellow fever virus, specific antigen, hepatitis B virus, HIV-1 succinylacetone, sulfadoxine, theophylline, thyrotropin (TSH), thyroxine (T4), thyroxine-binding globulin, transferrin, UDP-galactose-4-epimerase, urea, uroporphyrinogen I synthase, vitamin A, white blood cell, zinc protoporphyrin, salt, sugar, protein, fat, vitamin, etc).

An analyte also can comprise a contrast agent for imaging, a radioisotope, a chemical agent, fluorocarbon-based synthetic blood, etc.

The term "analyte" designates without limitation a substance or chemical constituent in a biological fluid (for example, blood, interstitial fluid, cerebral spinal fluid, lymph fluid or urine) that can be analyzed. Analyte can include naturally occurring substances, artificial substances, metabolites, and/or reaction products. Contemplated analytes include but are not limited to acarboxyprothrombin; acylcarnitine; adenine phosphoribosyl transferase; adenosine deaminase; albumin; alpha-fetoprotein; amino acid profiles (arginine (Krebs cycle), histidineurocanic acid, homocysteine, phenylalaninetyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, cystic fibrosis, DuchenneBecker muscular dystrophy, glucose-6-phosphate dehydrogenase, hemoglobin A, hemoglobin S, hemoglobin C, hemoglobin D, hemoglobin E, hemoglobin F, D-Punjab, beta-thalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheriatetanus antitoxin; erythrocyte arginase; erythrocyte protoporphyrin; esterase D; fatty acidsacylglycines; free β-human chorionic gonadotropin; free erythrocyte porphyrin; free thyroxine (FT4); free tri-iodothyronine (FT3); fumarylacetoacetase; galactosegal-1-phosphate; galactose-1-phosphate uridyltransferase; gentamicin; glucose-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), BA-1, β); lysozyme; mefloquine; netilmicin; phenobarbitone; phenytoin; phytanicpristanic acid; progesterone; prolactin; prolidase; purine nucleoside phosphorylase; quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measlesmumpsrubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzirangeli, vesicular stomatis virus, Wuchereria bancrofti, yellow fever virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins and hormones naturally occurring in blood or interstitial fluids can also constitute analytes in certain embodiments. The analyte can be naturally present in the biological fluid, for example, a metabolic product, a hormone, an antigen, an antibody, and the like. Alternatively, the analyte can be introduced into the body, for example, a contrast agent for imaging, a radioisotope, a chemical agent, a fluorocarbon-based synthetic blood, or a dose or pharmaceutical composition, including but not limited to insulin; ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbituates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer doses (analogs of fentanyl, meperidine,. amphetamines, methamphetamines, and phencyclidine, for example, Ecstasy); anabolic steroids; and nicotine. The metabolic products of doses and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-dihydroxyphenylacetic acid (DOPAC), homovanillic acid (HVA), 5-hydroxytryptamine (5HT), and 5-hydroxyindoleacetic acid (FHIAA).

The frequency of measurements can be variable, for example contextual (e.g. repeated if a risk of hypoglycemia is higher than a predefined threshold), can depend on the current uncertainty of associated algorithms.

In some embodiments of the invention, the system comprises a monitoring device responsible for the detection of a particular analyte. The sensing region generally comprises a nonconductive body, a working electrode (anode), a reference electrode (optional), and/or a counter electrode (cathode) passing through and secured within the body forming electrochemically reactive surfaces on the body and an electronic connective means at another location on the body, and a multi-domain membrane affixed to the body and covering the electrochemically reactive surface.

### Hardware

Computing and storage resources of the system can be locally accessed and/or accessed from locations in the "cloud" (in one or more servers accessible by one or more communication channels). A local system, e.g. an electromagnetic dose delivery device, can comprise the core medical features (emitter, source of energy, emitter, antennas). It may further comprise communication capabilities (according to continuous or episodic or intermittent or even opportunistic modes). In other words, a computer (processor) and storage (memory unit) can be remotely accessed. The rendering of the data to be displayed may be handled in the cloud.

A local display device can then act as a display device (i.e. the images being communicated to the local display device can be uncompressed and do not need local processing steps).

In an embodiment, accessories can host one or more media, for example stored in a micro-SD card (with video or audio tutorials accessible to a computer nearby, in order to help a patient to use the system according to the invention.

In some embodiments, the architecture of the system can be modular (different parts can be connected, and individually replaced). In an embodiment, each part of the architecture can broadcast service messages and the global system can be coordinated. In some embodiments, some parts can be 3D printed if not bio-printed.

Association or pairing between apps can use QR codes, or barcodes, or tokens, or communication protocols, with or without the intervention of a user. The disconnection of one or more parts can trigger an alarm.

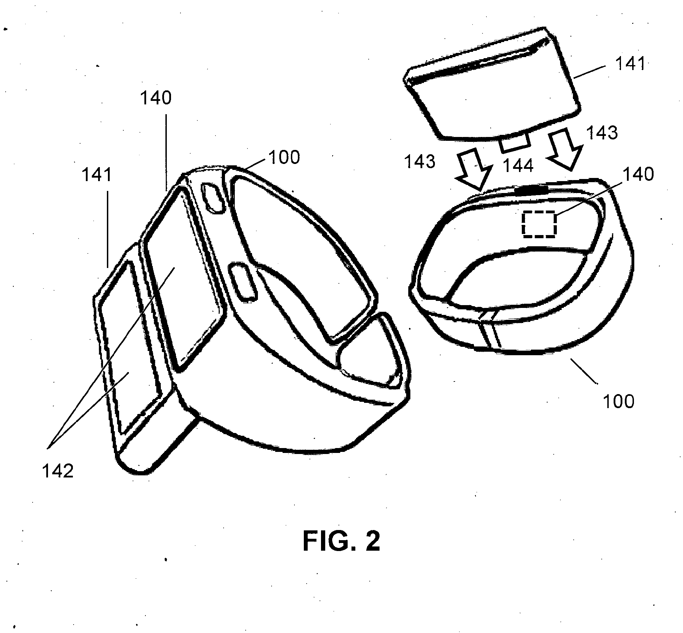

FIG. 2 illustrates an embodiment of the system according to the invention.

In an embodiment, the medical system 100 is arranged in an armband housing. The medical system comprises the millimetric emitter 110, a logic circuit 120, optional sensors 130 and a battery or (first) energy source 140 (indicated in dotted line).

In an embodiment, the first energy source 140 is rechargeable by wired USB or wirelessly e.g. by induction).

In an embodiment, the first energy source is a low-capacity rechargeable battery which is used to power on internal circuits (e.g. Bluetooth BLE communication, the logic circuit and optional sensors).

In an embodiment, the medical system 100 and thus the (first) energy source 140 is connectible (can be connected) to a second energy source 141.

In an embodiment, the second energy source 141 is releasable.

In an embodiment, the second energy source 141 is used to recharge the first energy source 140 (but the latter can yet be recharged independently).

The second energy source can vary in capacity (e.g. 100 mAh, 200 mAh, 400 mAh, 800mAh etc).

In some embodiments, the user can choose between different second energy sources, which serve to inject electromagnetic radiations, also designated as "doses". In other words, a second energy source 141 can be used to power on the millimetric emitter module. As long as the dose is delivered, the battery remains affixed to the medical system 100. When the second energy source is empty, or when the desired dose is delivered, said second energy source can be removed or released.

In an embodiment, a second energy source 141 is rechargeable.

Two-part battery embodiments are advantageous in that the medical system worn by the user remains minimal in size; the "footprint" or total size of the device (armband plus second energy source) is minimized, while ensuring that the emitter is continuously powered on and/or that data stemming out of the optional sensors is well received.

As a further advantage, the use of a dedicated energy source for e.m. radiation lets the user of the medical device can inject a plurality of doses, for example in a sequence.

As a further advantage, other units of second energy sources can be recharged in parallel, for example during a dose delivery.

As a further advantage, the first energy source can be incidentally or opportunistically recharged by the second energy source (thereby simplifying the energy management of the medical device.

The capacity of a second energy source 141 can be indicated in diverse ways: sizes of the housing oft the second energy source can be discretized or quantized (discrete volumes and/or weights). Indications of capacity can be labeled or otherwise indicated (e.g. color-codes, QR codes, etc). Different colors can indeed be associated with different doses.

In an embodiment, a second energy source 141 comprises or is associated with a diode, indicating various colors corresponding to various recharging actual states and/or capacities.

In an embodiment, a second energy source 141 comprises or is associated with a display 142 or screen, displaying predefined colors indicative of the actual and/or maximal (or nominal) charge.

For example, an empty second energy source 141 can be indicated by a red or absent color (upon move or gesture or test button triggering). A full second energy source 141 can be indicated by a green color.

In an embodiment, a second energy source can be associated with information or data associated with dose delivery. For example, the choice of a particular capacity amongst many can lead to specific dose deliveries (duration, electric power, etc)

### Associations system 100 and battery 140

In an embodiment, the association between the system 100 (in form of a wristband in this example) and the second battery 140 can be performed with a USB plug/port 144 (for example USB-C which port is symmetrical). One single point of contact can be sufficient to maintain a stable relation. In an embodiment, the plug and/or port are rotatable, so the connection is slightly flexible (which configuration can maintain a permanent contact).

USB-C connections are advantageous: with reversible plugs, it can be plugged into a device any which way; it can carry up to 100W of charging power (from 5V/0.5A up to 20V/5.0A) and it is able to merge both legacy Type-A and -B functions in a single port and can even change roles on the fly. Devices with a USB-C port recharge quickly and offer ultra-fast transfer speeds. USB Type-C can be associated with power delivery controllers. Such controllers can be integrated in the battery 140 for example (or in the fitness band). These controllers can handle current negotiation between source and sink according to standardized protocols.

In an embodiment, the association between the system 100 and the second battery 140 is obtained by one or more magnets 143. In practice, a pair of magnets allows stable connection, while energy transfer can be enabled at the same time (e.g. pin magnetic connectors, male and female spring pin magnetic connectors, pogo pin magnet connectors, etc). In addition to conductivity, data transfers can occur indeed.

In some embodiments, the battery 140 can comprise a ROM and/or RAM memory, or more generally, volatile and/or non-volatile memory. Such memories can be used to backup data, or to indicate a programmed radiation dose. In addition or in substitution of memory units, the battery can comprise or be associated with a RFID or NFC tag. Such tag can be programmable and programmed. The wristband can then comprise a tag reader, configured to determine the radiation dose as it has been programmed.

In some embodiments, the association between the system 100 and the battery 140 can comprise protection mechanisms from reverse polarity.

Other embodiments are now described.

There is disclosed a system 100 comprising a millimeter waves emitter 110, with one or more frequencies selected in the band 61.0 - 61.5 GHz and a power flux density of approximately 10 mW/cm², connectable to a source of energy 140.

The power can range from 5mW/cm2 to 15mW/cm2. A power flux density of at least 0.5 milliwatts per square centimeter of surface can allow the treatment to be effective and to reduce pain. The power flux density can be configurable.

The mentioned frequency ISM band is particularly effective for the treatment by millimeter waves. The ISM radio bands are portions of the radio spectrum reserved internationally for industrial, scientific and medical (ISM) purposes. In some embodiments, the optimal effect of a treatment by millimeter waves can be obtained with a frequency around 61.25 GHz and a power flux density of approximately 10 mW/cm2.

Regarding the aptitude to expose at least 2.5 square centimeters of surface, the system can, for example, present a module comprising several antennas transmitting waves simultaneously, the area covered by all the antennas, and therefore by the module, can represent at least 2.5 square centimeters exposed to waves. Such a surface can sufficient for the wrist, but for other less innervated areas it could be advantageous to expose larger surfaces (ankle for example, wherein the emitter can be embedded in a shoe).

In an embodiment, the source of energy can comprise one or more batteries.

In some embodiments, the emitter can be "batteryless", e.g. directly associated with electrical outlet, or energy beaming. For example, a USB port (e.g. micro-USB or USB-C) can be used to deliver energy directly to the emitter.

In an embodiment, there can be one single battery (e.g. the source of energy 140).

In an embodiment, there can be two energy sources or batteries, which can be charged independently from each other.

In an embodiment, the energy source or battery 140 is low-capacity and is recharged by a large-capacity second battery 141.

In a development, the system 100 further comprises a logic circuit 120 and one or more sensors and/or actuators 130 associated with the millimeter waves emitter 110 and the energy source 140.

In an embodiment, the logic circuit and/or one or more sensors and/or the energy source 130 are associated with (or connected to, or interconnected to, or regulating, or controlling) the millimeter waves emitter 110

The logic circuit in particular can comprise processing and memory means or elements or units or circuit blocks. In an embodiment, the logic circuit further comprises a communication circuit.

The one or more sensors and/or actuators 130 can comprise sensors (e.g. proximity sensor) and/or actuators (e.g. proximity sensor and/or buzzer or vibratile actuator).

An actuator for example can be a buzzer (vibratile element), a heat sink, an inflatable part to maximize the contact between skin and emitter, a skin or tissue massage mechanism via electro-mechanical miniaturized devices, etc.

In an embodiment, the system is embedded in a housing (e.g. armband, necklace, shoe, etc), for example configured to place the emitter at the proximity of the skin of a mammal.

In an embodiment, the energy source 140 is embedded in a housing (e.g. armband, necklace, shoe): it can be permanently affixed or mounted.

In a development, the system further comprises a second energy source 141 connected to one or more parts of the system 100.

For example, the second energy source 141 can be connected to the (first) energy source 140 and/or to the emitter 110. The energy source 140 can be embedded in a housing comprising the emitter and other parts, and the second energy source 141 can be releasable.

In an embodiment, the second energy source is connectible to the emitter, for delivering radiation doses.

In a development, the energy source 141 is releasable.

In an embodiment, the connection between the emitter and batteries can be permanent (or tied up, or imprisoned), but in some embodiments, the source of energy can be disconnected (or set free, allowed to leave, liberated, released). This advantageous form factor enables more lightweight radiation devices.

In an embodiment, in addition to a releasable source of energy, the latter source of energy 141 can be disposable. Advantageously, batteries available in the market can be used.

In some other embodiments, the source of energy can be rechargeable. For example, a battery of type CR2032 is a disposable battery while ML2032, VL2032 and LIR2032 types are rechargeable. An alternative to rechargeable batteries for electricity storage is supercapacitors, now being used in some devices such as the mechanically powered flashlight.

A primary cell (disposable) is a battery (a galvanic cell) that is designed to be used once and discarded, and not recharged with electricity and reused like a secondary cell (rechargeable battery): alkaline, aluminum-air, Bunsen, Chromic acid, Clark, Daniell, Dry, Edison-Lalande, Grove, Lithium, Lithium-air, Metal-air battery, oxyhydroxide, Silicon-air, silver oxide, Weston, Zamboni, Zinc-air, Zinc-carbon.

Secondary cell (rechargeable) can comprise one or more of: lead-acid , gel / VRLA, Lithium-air, Lithium ion , Lithium polymer, Lithium iron, Lithium titanate, Lithium-sulfur, Dual carbon, Metal-air battery, Molten salt, Nanopore, Nanowire, Nickel-cadmium, Nickel-hydrogen, Nickel-iron, Nickel-lithium, Nickel-metal hydride, Nickel-zinc, Polysulfide bromide, Potassium ion, Rechargeable alkaline, Silver zinc, Sodium ion, ,Sodium-sulfur ,Solid-state, Vanadium redox, Zinc-bromine, Zinc-cerium.

In a development, the second energy source 141 comprises one or more of a diode and/or screen.

A lighting system, for example LED diode or a plurality thereof forming a screen can be formed. Advantageously, information can be conveyed using said lighting (about the status of the battery, for example available charge or programmed radiation dose).

In a development, the connection between the emitter 110 and/or an energy source 140 and/or 141 is wireless, e.g. made by power beaming.

Wireless power transfer (WPT), wireless power transmission, wireless energy transmission (WET), or electromagnetic power transfer is the transmission of electrical energy without wires as a physical link.

Wireless power techniques comprise near field (using inductive coupling between coils of wire, or by electric fields using capacitive coupling between metal electrodes) and far-field transmission (or "radiative techniques", also called "power beaming", power is transferred by beams of electromagnetic radiation, like microwaves or laser beams. These techniques can transport energy, aiming the receiver; therefore object tracking can be used. Power transmission via radio waves can be made directional. A rectenna may be used to convert the microwave energy back into electricity. A rectenna (rectifying antenna) is a special type of receiving antenna that is used for converting electromagnetic energy into direct current (DC) electricity.

Under experimental conditions, microwave conversion efficiency was measured to be around 54% across one meter. Wireless power transmission using phased array antennas can deliver electrical power up to 30 feet. Wi-Fi can be used to wirelessly trickle-charge nickel-metal hydride and lithium-ion coin-cell batteries at distances of up to 28 feet.

Wireless energy transfer systems using lasers for consumer electronics can be used. For example, the laser light can also be guided by an optical fiber ("power-over-fiber" technology) In other embodiments, a wireless charging system can work via ultrasound. In an embodiment, far-field wireless power transfer can use focused infrared beams (the system consists of a transmitter and a receiver; transmitter connects to a standard power outlet and converts electricity into infrared laser beam; receivers use a miniature photo-voltaic cell to convert transmitted light into electrical power).

In a development, the system further comprises an object tracking device or mechanism configured to control said power beaming.

A'tracking system or locating system/mechanisms can indeed be used for the observing of an object on the move and supplying a timely ordered sequence of location data for further processing.

Motion capture (sometimes referred as mo-cap or mocap, for short) designates the process of recording the movement of objects or people. Object tracking can be performed using various techniques. Optical systems tracking system comprise passive markers and/or active markers, time modulated active marker, semi-passive imperceptible marker or marker less. Non-optical systems comprise inertial systems, mechanical motion, magnetic systems or stretch sensors. Optical systems and non-optical systems can be combined. Related techniques can also apply (e.g. RF positioning). On other embodiments, tracking can use vision-based trajectory tracking, inertial mechanisms, multilateration, real-time locating, track and trace, or a combination thereof.

Communications/control between power source and target device can be performed in various ways. In an embodiment, the power source embeds an object tracking device, which beams energy to the (passive) millimeter waves emitter. In an embodiment, the object tracking device is embedded in the millimeter waves emitter (e.g. position in space of the millimeter waves emitter is communicated to the beaming power source, which adapts to this position based on said information). In an embodiment, the object tracking mechanism is distributed over the beaming power source and the millimeter waves emitter (for example cooperative tracking capability).

In a development, the type of an energy source is one or more of a cylindrical cell, a prismatic cell, a pouch cell, a CMS type battery or a combination thereof.

In some embodiments, a source of a battery can support one or more of types AAA, AAAA, A23, PP3, CR2032 and/or LR44 battery.

In a development, one or more of the emitter 110, the source of energy 140, the source of energy 141 is connectible to the battery of a consumer electronics device.

A consumer electronics device can be a computer, a laptop, a smartphone, a smartwatch, a tablet, a pair of earbuds, a VR helmet, AR glasses or other wearable devices. Such devices available at proximity of the user can be used opportunistically for dose radiation indeed. For example, a USB cable can drain energy from a consumer electronics device to the wristband. For example, energy stemming from smartphone can be directed towards the emitter and deliver a radiation dose. Such arrangement can imply that it can be sufficient for the user to carry on an emitter "naked", with a minimal number of associated components, if energy available in the vicinity of the user can be used.

Such arrangement may imply the consumer electronics device e.g. smartphone is maintained in the vicinity of the wristband and emitter, but this temporary arrangement can be eased in various ways (for example by placing a smartphone in an armband). Fast charge also can ease the drain of energy.

In some embodiments, consumer electronics device can comprise replaceable batteries. Such batteries can thus be modified to integrate the system as described. In such a case, the smartphone may carry the described system: the user willing to radiate a dose can maintain the carrying device in contact to the skin. In practice, the smartphone can lay on top of a table and the user can lay down (or apply or pose) his forearm on top of the smartphone, so that his wrist touches the indicated placement of the emitter embedded in the smartphone.

In a development, the control of the emitter and/or the energy source can be configured according to one or more energy management schemes, including light or deep hibernations.

Electronic circuits selectively can be powered-off (for example according to criticity levels associated with the different electronic circuits constituting the system). Various cooling off systems can be used

Energy management schemes can handle the recharging of energy sources 140 and/or 141. For example, the energy source 140 can be associated with a higher priority for recharge than the energy source 141. The energy source 141 can be used to perform radiation dose delivery, but used to recharge at first the internal energy source 140, powering on the electronic circuits 120 and/or sensors/actuators 130.

In a development, one more sensors 130 are configured to detect the presence and/or concentration of one or more predefined biomarkers.

In biomedical contexts, a biomarker (or biological marker) is a measurable indicator of one or more biological states (or conditions). Biomarkers are often measured and evaluated using blood, urine, or soft tissues to examine normal biological or pathogenic processes, or responses to a therapy. So called "digital biomarkers can be collected by biosensors (e.g. accelerometer, heartrate, on-skin sweat analysis (sudorology). Various therapy approaches can be developed using artificial intelligence techniques (machine learning). Biomarkers can be classified in molecular biomarkers, cellular biomarkers or imaging biomarkers. These biomarkers can present a clinical role in narrowing or guiding treatment decisions. Biomarkers can be either predictive, prognostic, or diagnostic. Predictive biomarkers can serve predicting clinical outcomes, e.g. help optimize ideal treatments. Predictive biomarkers are genes such as ER, PR and HER2/neu in breast cancer, BCR-ABL fusion protein in chronic myeloid leukaemia, c-KIT mutations in GIST tumours and EGFR1 mutations in NSCLC. Diagnostic biomarkers can serve a role in narrowing down diagnosis. For example, after a heart attack a number of different cardiac biomarkers can be measured. More generally, a biomarker can be a traceable substance enabling to examine functions or other aspects of health. It can be a substance whose detection indicates a particular disease or infection. A biomarker can indicate a change in expression or state of a protein that correlates with the risk or progression of a disease (e.g. prostate-specific antigen). A prognostic biomarker can provide information about overall outcome, regardless of therapy.

In a development, the system further comprises a logic circuit 120, wherein said logic circuit 120 is configured to control the emitter 110 depending on the presence and/or concentration of said one or more predefined biomarkers.

Control can be local indeed (i.e. logic controlling emitter based on sensors' data).

In an embodiment, the control of the medical system is configured according to the presence and/or concentration of one or more predefined biomarkers and/or bioindicators.

Likewise, one or more bioindicators can be monitored in the environment for changes (biochemical, physiological, or behavioral). High voice volumes, background noises, repeated movements or the lack thereof, can constitute bioindicators that can contribute estimating the stress or pain endured (e.g. over time). The presence and/or concentration (at a given time or taken into account evolution dynamic thereof) can lead to appropriately modulate

Methods of discovery comprise different possible approaches (i.e. genomics, proteomics, metabolomics, lipidomics, glycomics, etc). Bioindicators can comprise microbial indicators (e.g. chemical pollutants, microalgae).

In a development, a source of energy 140 or 141 comprises lithium-ion, lithium-iron, lithium-sulfur, solid-state, graphene, or combination thereof.

In some embodiments, hand cranked dynamos can be used (or hand powered flashlights and other human powered equipment to recharge batteries. Human-powered equipment primarily consists of electrical appliances which can be powered by electricity generated by human muscle power as an alternative to conventional sources-of electricity such as disposable primary batteries and the electrical grid. Such devices contain electric generators or an induction system to recharge their batteries. Separate crank-operated generators are now available to recharge battery-powered portable electronic devices such as mobile phones. Others, such as mechanically powered flashlights, have the generator integrated within the device. Wrist watches can use muscle power to keep their mainsprings wound up.

In a development, a source of energy 140 or 141 comprises a fast charge battery.

The expression "fast charge" designates battery charging protocol(s) used for managing power delivered over USB or other means, mainly by communicating to the power supply and negotiating a voltage. Existing techniques allow to prevent the battery damage caused by uncontrolled fast charging and regulating the incoming voltage internally. Wall adaptors, or in-car chargers, or power banks can be used to deliver charge.

In an embodiment, the system is being configured to be attached to an animal.

Mammals present analogue physiological traits. The described systems can advantageously be used for farming, livestock farming, cattle farming, sheep farming, pig farming, poultry farming. Animals which can bear such devices can be cows, livestock's, chickens. The well-being of such animals can be improved and thus the production can be improved. Domestic companion animals such as dogs and cats also can wear such systems, for example for recovery or when pain is likely to happen for a reason or another.

In a development, the system communicates with another electronic device and/or a distant server, via one or more networks.

Another electronic device can be a PC, a smartphone, a smartwatch or a combination thereof.

Networks can be short-range communications and/or Internet of Things networks (Sigfox, LoRa)

In some embodiments, the system or parts thereof are arranged and/or configured according to one or more communication schemes.

Various communications (e.g. WIFI, Bluetooth, LoRa, Sigfox etc.), protocols, modulations (e.g. CDMA), medium/media (e.g. wired/wireless) or data transport schemes can be used.

Communications can use a plurality of networks, comprising NFC, beacon, Wi-Fi, Li-Fi, WiMAX, 2G, 3G, 4G and 5G.

The different devices and/or sensors can use a diversity of communications schemes and/or networks topology (e.g. peer-to-peer, mesh, ad hoc, centralized, etc) and/or technology

### (Bluetooth Low Energy BLE, WIFI, Li-Fi, beacon, etc)

In some embodiments, the system can be part of a mesh or ad hoc network (loosely coupled devices, offering ephemeral controllability of the global system).

Communications can use any of a plurality of communications standards, protocols and technologies, including but not limited to Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (Wi-Fi) (e.g., IEEE 802.11a, IEEE 802.11ac, IEEE 802.11ax, IEEE 802.11b, IEEE 802.11g and/or IEEE 802.11n), voice over Internet Protocol (VoIP), Wi-MAX, a protocol for e-mail (e.g., Internet message access protocol (IMAP) and/or post office protocol (POP)), instant messaging (e.g., extensible messaging and presence protocol (XMPP), Session Initiation Protocol for Instant Messaging and Presence Leveraging Extensions (SIMPLE), Instant Messaging and Presence Service (IMPS)), and/or Short Message Service (SMS). In some embodiments, communications can use Zigbee, Ant, IrDa, audio or a combination thereof.

The present document is not a medical advice.

When lists of elements are provided, it is intended that-even if forgotten i.e. not explicitly mentioned-, combinations of such elements are possible. The expression "and/or" is generally applicable to this entire document; for example a sentence such as "the device can comprise element A, element B and element C" should be interpreted as "the device can comprise element A and/or element B and/or element C", i.e. the device comprises "A and B and C", or "A and B", or "A and C", or "B and C", "A", or "B", or "C".

A plurality of "incentives to combine" is now provided. It is suggested to the reader or skilled person to combine any of the presently described embodiments with one another.

It is also suggested to combine described embodiments or combinations thereof with one or more of the following words, suggesting techniques or technologies or concepts or ideas or paradigms or states or the like: abdomen (e.g. for attachment or implantation), abrasion (e.g. skin local laser abrasion for access to subcutaneous or capillary or interstitial blood), acoustic (e.g. audio monitoring for pain estimation, or waves used for massaging of irradiated tissues), activity (monitoring, e.g. running, sleeping, etc), adaptive (e.g. depending on rules), adhesive (e.g. the emitter can be attached by adhesive e.g. glue), ankle (e.g. as a possible radiation location), arm (e.g. band to attach the emitter), attention (monitoring, e.g. eye tracking which can measure the level of stress and/or well-being), belt (e.g. belt supporting the moving emitter for automated irradiations), biochips (e.g. to measure biomarkers, bioindicators, etc), biofeedback (e.g. to adjust radiations), capillary (e.g. capillary blood measures to adjust radiations), cardiograms (e.g. to adjust radiations), cerebral measures, cardiac coherence (to be used in combination with radiations by the emitter), connected emitter (e.g. to other connected devices), counter (e.g. independent mechanism for safety, maximal amount or radiation), dental and dentistry (e.g. emitter can be implanted in a teeth, with charging by induction), display (e.g. the emitter can be associated with one or more displays), electrocardiograph or electroencephalograph or encephalograph (e.g. which can be associated with the emitter), expandable or expansible (e.g. for example of a bladder configured to adjust the distance between emitter and skin), seat fasteners (integration within to deliver doses while driving), fingerprinting (e.g. a biometric measures can be required to upload data associated with doses; for example a docking station for battery recharge can be associated with a fingerprinting, which data can be required to recharge and otherwise upload data, thus authenticated), face analysis (e.g. masticatory muscle and/or facial expressions which can provides clues for occurrence of pain. It provides an important window for the person who cannot verbally describe or rate their level of pain), fuzzy logic (e.g. which can be used to deliver doses), galvanic current (e.g. to contribute estimating pain), gloves (the emitter can be mounted in a glove, wherein radiation is distributed over skin surface), GPS (e.g. delivery of doses can be based on rules depending on parameters such as geolocation), headphones (e.g. headphones can integrate one or more emitters), helmet (e.g. emitter can be integrated into a headset), housing (e.g. emitter can be integrated into a housing comprising the emitter, a support for energy source and headset and communication electronics), induction (e.g. emitter can be powered by induction), knee (e.g. possible location for radiation), masks (e.g. emitter's antennas can be covered by one or more masks, so as to change the waveguide, e.g. electromagnetic lobes), safety mechanism (e.g. emitter can be controlled by an independent circuit that prevents overdosing), microneedles (e.g. the radiation can be rendered minimally invasive, for example if guided by such microneedles), modular design (e.g. the system can be modular, e.g. comprising a battery holders of different types being connectable to the emitter, therefore presenting flexibility for dose delivery), modulation (e.g. dose deliveries can be modulated in various ways), mounted (e.g. emitter can be integrated with different mounting systems), neck (e.g. emitter can be placed on a necklace), pattern (e.g. doses can be delivered according to one or more patterns), piercing (e.g. emitter can be integrated into a piercing), pneumatic (e.g. one or more pneumatic mechanisms can be used to adjust the distance between the emitter and the skin of the user), posture (e.g. gestures of the user can be captured and can contribute estimating pain), pulses (e.g. emitter can be used to deliver pulsed doses), repositioning (e.g. the emitter can be repositioned in predefined positions), respiratory (e.g. respiratory data can be captured and can contribute estimating pain, in turn doses delivery), saliva (e.g. saliva testing can be particularly useful for performing chronobiology assessments), shields (as for masks, one or more shields can be used, to modify electromagnetic deliveries), slide to unlock (e.g. safety can use different manual or mechanical manipulations before activation), strap (e.g. emitter can be integrated into a strap), waveform and wavelengths (e.g. emitter delivery doses can be varied in terms of waveforms and in particular wavelengths), wrist and wristwatch (e.g. emitter can be integrated into a wristwatch).

## Claims

1. A system 100 comprising a millimeter waves emitter 110, with one or more frequencies selected in the band 61.0 - 61.5 GHz and a power flux density of approximately 10 mW/cm², connectable to a source of energy 140.

2. The system of Claim 1, wherein the system 100 further comprises a logic circuit 120 and. one or more sensors and/or actuators 130 associated with the millimeter waves emitter 110 and the energy source 140.

3. The system of any preceding Claim, further comprising a second energy source 141 connected to one or more parts of the system 100.

4. The system of Claim 3, wherein the energy source 141 is releasable.

5. The system Claim 3, wherein the second energy source 141 comprises one or more of a diode and/or screen.

6. The system of any preceding Claim, wherein the connection between the emitter 110 and/or an energy source 140 and/or 141 is wireless, e.g. made by power beaming.

7. The system of Claim 6, further comprising an object tracking device or mechanism configured to control said power beaming.

8. The system of any preceding Claim, wherein the type of an energy source is one or more of a cylindrical cell, a prismatic cell, a pouch cell, a CMS type battery or a combination thereof.

9. The system of any preceding Claim, wherein one or more of the emitter 110, the source of energy 140, the source of energy 141 is connectible to the battery of a consumer electronics device.

10. The system of any preceding Claim, wherein the control of the emitter and/or the energy source can be configured according to one or more energy management schemes, including light or deep hibernations.

11. The system of any preceding Claim, wherein one more sensors 130 are configured to detect the presence and/or concentration of one or more predefined biomarkers.

12. The system of Claim 11, further comprising a logic circuit 120, wherein said logic circuit 120 is configured to control the emitter 110 depending on the presence and/or concentration of said one or more predefined biomarkers.

13. The system of any preceding Claim, wherein a source of energy 140 or 141 comprises lithium-ion, lithium-iron, lithium-sulfur, solid-state, graphene, or combination thereof.

14. The system of any preceding claim, wherein a source of energy 140 or 141 comprises a fast charge battery.

15. The system of any preceding claim, wherein said system communicates with another electronic device and/or a distant server, via one or more networks.
